(19) Europäisches Patentamt
European Patent Office
Office européen des brevets

(11) **EP 2 155 229 B1**

(12) **EUROPEAN PATENT SPECIFICATION**

(45) Date of publication and mention
of the grant of the patent:
**19.01.2011   Bulletin 2011/03**

(51) Int Cl.:
*A61K 38/11* (2006.01)     *A61K 38/21* (2006.01)
*A61K 38/22* (2006.01)     *A61K 38/23* (2006.01)
*A61K 38/26* (2006.01)     *A61K 38/28* (2006.01)
*A61K 38/29* (2006.01)     *A61K 31/575* (2006.01)
*A61K 9/46* (2006.01)      *A61K 9/14* (2006.01)
*A61K 9/08* (2006.01)      *A61K 38/00* (2006.01)

(21) Application number: **08754187.6**

(22) Date of filing: **01.05.2008**

(86) International application number:
**PCT/US2008/005655**

(87) International publication number:
**WO 2008/137054 (13.11.2008 Gazette 2008/46)**

(54) **COMPOSITION FOR TRANSMUCOSAL DELIVERY OF POLYPEPTIDES**

ZUSAMMENSETZUNG ZUR TRANSMUKOSALEN ABGABE VON POLYPEPTIDEN

COMPOSITION POUR L'ADMINISTRATION TRANSMUQUEUSE DE POLYPEPTIDES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority:  **01.05.2007   US 927006 P**

(43) Date of publication of application:
**24.02.2010   Bulletin 2010/08**

(73) Proprietor: **Cephalon, Inc.**
**Frazer, PA 19355 (US)**

(72) Inventors:
 • **DURFEE, Steve, L.**
  **Sandy, UT 84092 (US)**
 • **THURMAN, Gary, B.**
  **Sandy, UT 84094 (US)**

(74) Representative: **Hallybone, Huw George**
**Carpmaels & Ransford**
**One Southampton Row**
**London**
**WC1B 5HA (GB)**

(56) References cited:
**WO-A-00/59423        US-A- 5 863 555**
**US-B1- 6 200 604**

 • **VEUILLEZ F ET AL: "Factors and strategies for improving buccal absorption of peptides" EUROPEAN JOURNAL OF PHARMACEUTICS AND BIOPHARMACEUTICS, ELSEVIER SCIENCE PUBLISHERS B.V., AMSTERDAM, NL, vol. 51, no. 2, 1 March 2001 (2001-03-01), pages 93-109, XP004257246 ISSN: 0939-6411**

**Description**

**FIELD OF THE INVENTION**

The invention relates to the fields of biotechnology and pharmaceutical chemistry. In particular, the invention pertains to oral transmucosal delivery of biologically active polypeptides.

**BACKGROUND OF THE INVENTION**

Pharmaceutically active polypeptides are known to be useful in the medical field in a variety of therapies. Advances in biotechnology have enabled large-scale production of natural and recombinant polypeptides for manufacturing products in the pharmaceutical industry. Some polypeptides have been known, however, as poor candidates for oral gastrointestinal administration route due to their susceptibility to decomposition in the digestive tract. Accordingly, polypeptide therapy has typically been performed via the parenteral route, *e.g.*, infusion or injection.

Transmucosal administration of drugs, including polypeptides, using dosage forms with compositions that enhance transmucosal absorption have been developed. For example, Igari et al. U.S. Patent Nos. 5,725,852 and 5,482,706 describe mucosal administration of polypeptides in conjunction with cytidine diphosphate choline. Another method for transmucosal delivery of polypeptides, for example, is described in Acharya et al., U.S. Patent No. 6,210,699, using unplasticized polyvinyl pyrrolidone as a mucoadhesive. Transvaginal absorption of polypeptides is described in Fujii et al., U.S. Patent No. 5,238,917, wherein the absorption promoter includes polyoxyethylenealkyphenyl ether and cholic acid.

Oral transmucosal absorption is well-known to be associated with certain advantages, such as self-administration capability, faster onset of plasma concentration and therapeutic effect, and avoidance of first pass metabolism of the active ingredient. Various dosage forms have been developed for oral transmucosal delivery of certain active ingredients across the mucosa, *e.g.*, sublingual, buccal, gingival, palatal, and esophageal mucosal tissues. Such dosage forms have been designed for rapid disintegration and high concentrations of the active ingredient to effectuate absorption in the oral cavity.

Oral transmucosal administration of polypeptides has also been explored. For example, buccal administration of polypeptides is described in Heiber et al., U.S. Patent Nos. 5,863,555 and 5,766,620, wherein glucagon-like insulinotropic polypeptide is administered buccally in conjunction with a permeation enhancer such as bile salts.

One oral dosage form specifically formulated for oral transmucosal absorption of certain opiates such as fentanyl, has been developed under the brand name FENTORA® utilizing the ORAVESCENT® technology (available from CIMA LABS, Inc., Eden Prairie, Minnesota). This technology has been described in U.S. Patent Nos. 6,200,604 and 6,974,590, for example, as well as U.S. Published Patent Application Nos. 2005/0169989 (Serial No. 1/026,132 filed December 30, 2004); 2005/0142197 (Serial No. 1/026,327 filed December 30, 2004); 2005/0142198 (Serial No. 1/027,353 filed December 30, 2004); and 2005/0163838 (Serial No. 11/026,759 filed December 30, 2004). This particular technology uses an excipient formulation containing a pH adjusting substance and an effervescent couple to facilitate transmucosal transport of active ingredient fentanyl citrate. Identification of other pharmaceutically active compounds that might benefit from the oral effervescent dosage form administration of ORAVESCENT®, however, is ongoing.

There exists a need in the pharmaceutical field for dosage forms that succeed and improve in the delivery of large molecules, including polypeptides, across the oral mucosal tissue. There is a further need for formulations that accomplish transmucosal delivery efficaciously for larger molecular structures, including polypeptides.

**SUMMARY OF THE INVENTION**

The invention provides a composition that enhances transmucosal absorption of polypeptides across oral mucosal tissue and provides relatively rapid efficacious therapeutic onset thereof. It has been discovered that a composition can be prepared that enhances transmucosal absorption of polypeptides giving therapeutic serum concentrations to the recipient. It has also been discovered that the combination of effervescent transmucosal dosage forms with bile salts such as (sodium) taurocholate can significantly enhance the absorption of polypeptides when formulated according to the invention. The combination of effervescent compositions and bile salts can have a synergistic effect on transport of polypeptides across the oral mucosa, thereby producing bioavailability results that are greater than the sum of their individual absorptive enhancement effects.

The invention provides a composition for oral transmucosal absorption comprising a biologically active polypeptide; a bile salt; and an effervescent excipient component comprising an effervescent couple. The composition can optionally further comprise a pH adjusting substance, *e.g.*, as part of the effervescent excipient component or as part of the oral Transmucosal composition. In some embodiments, the composition comprises a solid, oral dosage form, *e.g.*, a tablet.

The invention further provides a composition comprising: a biologically active polypeptide; a bile salt; and an effervescent excipient component comprising an effervescent couple for use in a method of enhancing transmucosal absorption of a biologically active polypeptide; the composition can optionally further comprise a pH adjusting substance.

The invention provides an oral trans mucosal composition comprising a therapeutically effective amount of insulin, an effervescent excipient component; and a bile salt for use in methods of treating diabetes in a recipient.

## BRIEF DESCRIPTION OF THE DRAWINGS

The invention is further illustrated by the following figures - none of which are to be construed as necessarily limiting the invention.

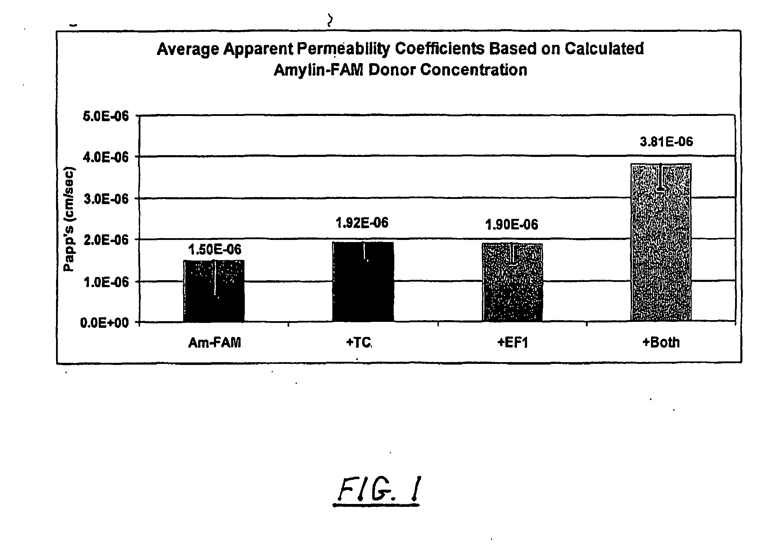

Figures 1 through 15 are bar charts showing comparative permeability of various polypeptides in combination with differing excipient compositions using *in vitro* epithelial cell culture tissue.

Figure 1 is a bar chart showing *in vitro* permeability of amylin by itself, in combination with a bile salt, in combination with an effervescent composition, and in combination with both bile salt and effervescent composition according to some embodiments of the invention. Fluorescently-labeled amylin is measured by fluorescence detection.

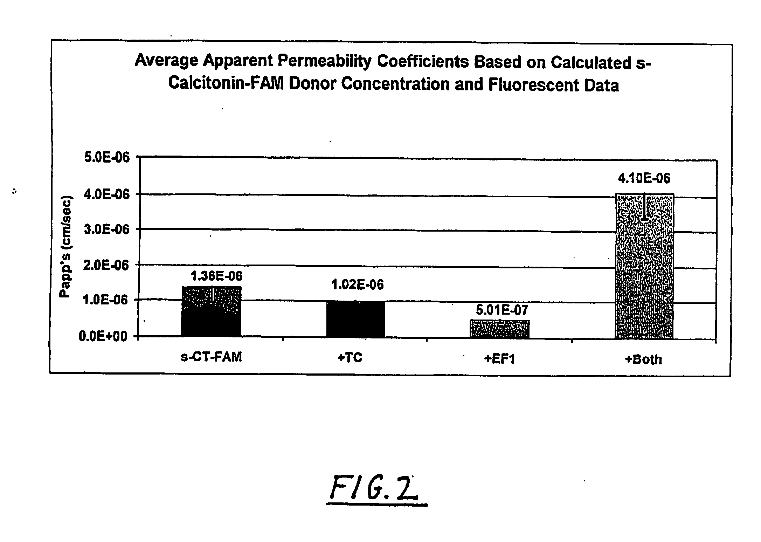

Figure 2 is a bar chart showing *in vitro* permeability of salmon calcitonin by itself, in combination with a bile salt, in combination with effervescent composition, and in combination with both bile salt and effervescent composition according to some embodiments of the invention. Fluorescently-labeled calcitonin is measured by fluorescence detection.

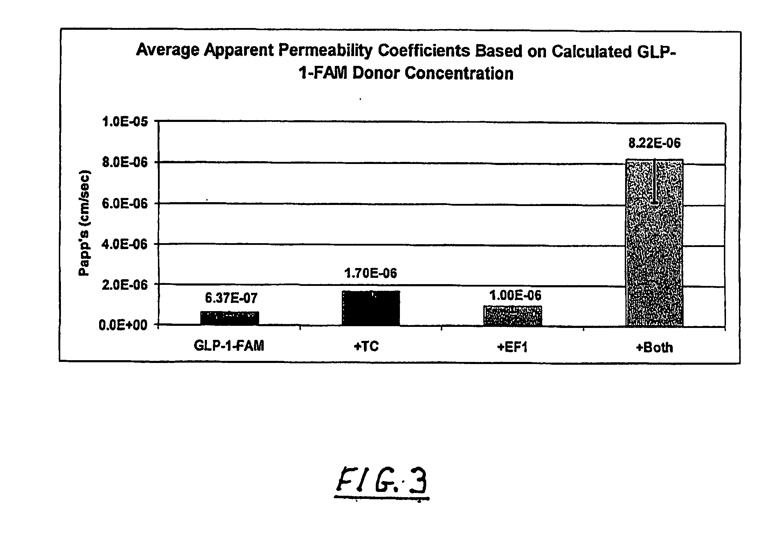

Figure 3 is a bar chart showing *in vitro* permeability of GLP-1 by itself, in combination with a bile salt, in combination with effervescent composition, and in combination with both bile salt and effervescent composition according to some embodiments of the invention. Fluorescently-labeled GLP-1 is measured by fluorescence detection.

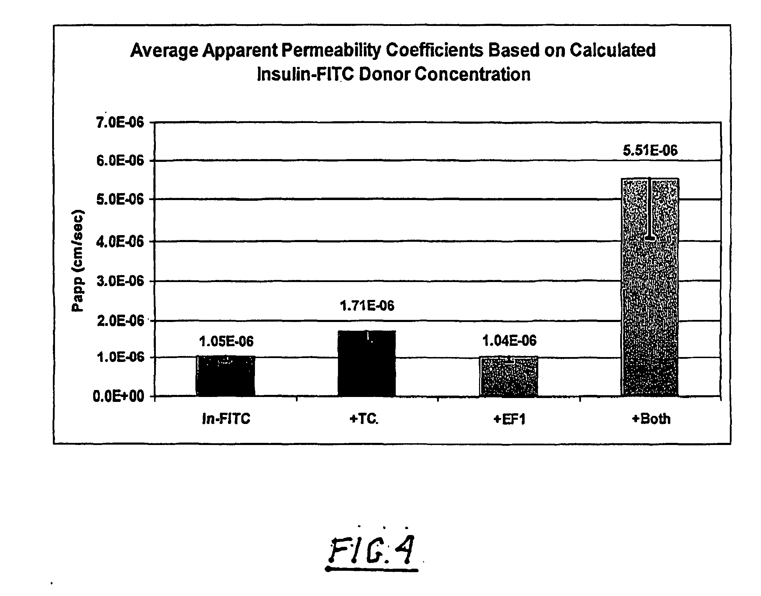

Figure 4 is a bar chart showing *in vitro* permeability of insulin by itself, in combination with a bile salt, in combination with effervescent composition, and in combination with both bile salt and effervescent composition according to some embodiments of the invention. Fluorescently-labeled insulin is measured by fluorescence detection.

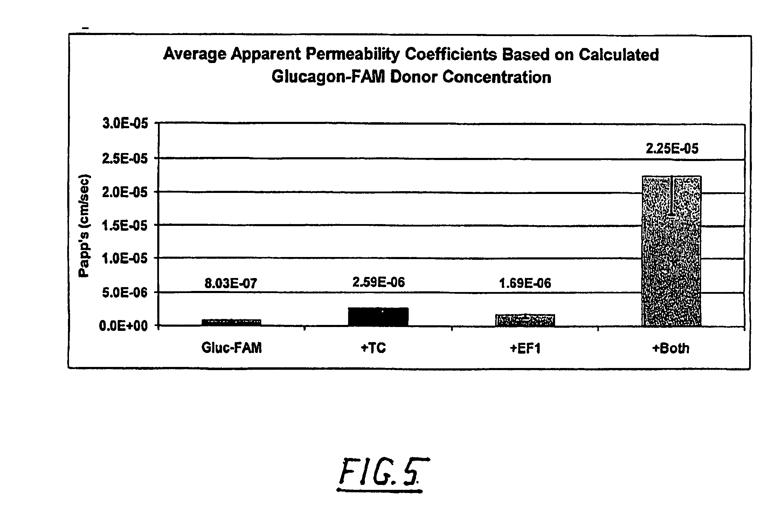

Figure 5 is a bar chart showing *in vitro* permeability of glucagon by itself, in combination with a bile salt, in combination with effervescent composition, and in combination with both bile salt and effervescent composition according to some embodiments of the invention. Fluorescently-labeled glucagon is measured by fluorescence detection.

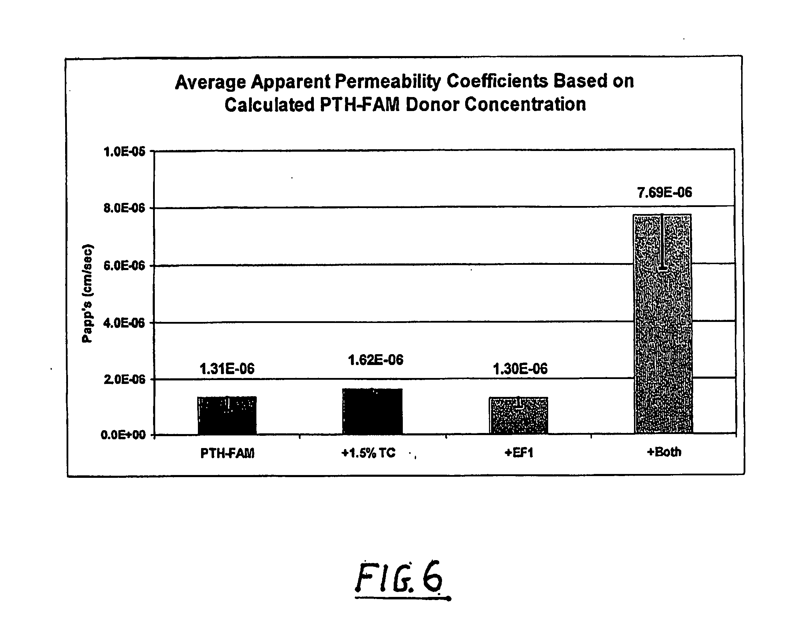

Figure 6 is a bar chart showing *in vitro* permeability of PTH by itself, in combination with a bile salt, in combination with effervescent composition, and in combination with both bile salt and effervescent composition according to some embodiments of the invention. Fluorescently-labeled PTH is measured by fluorescence detection.

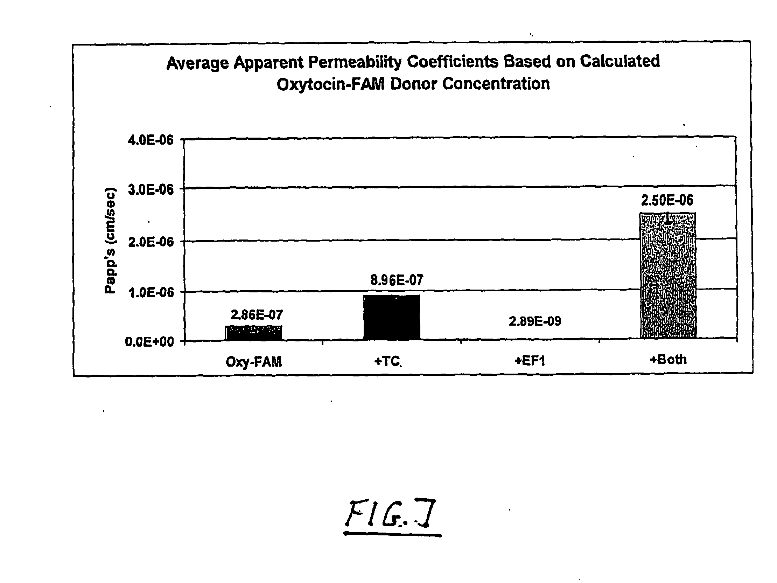

Figure 7 is a bar chart showing *in vitro* permeability of oxytocin by itself, in combination with a bile salt, in combination with effervescent composition, and in combination with both bile salt and effervescent composition according to some embodiments of the invention. Fluorescently-labeled oxytocin is measured by fluorescence detection.

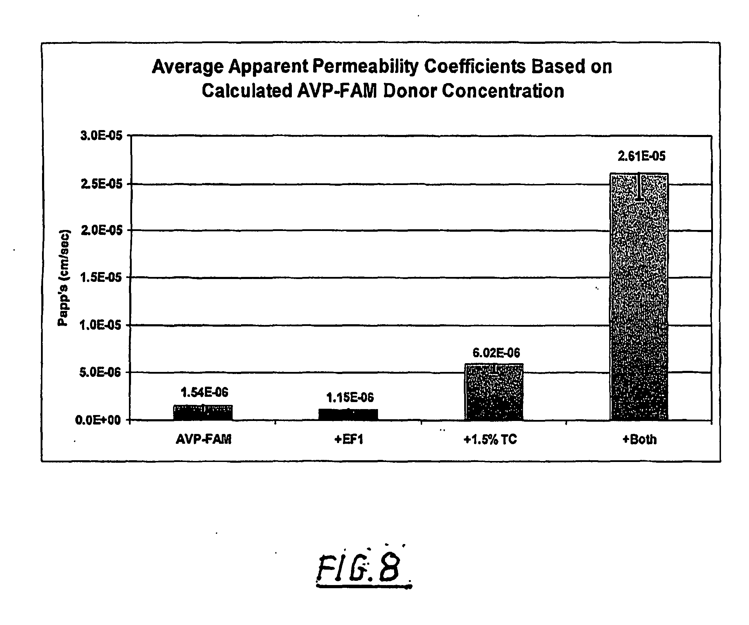

Figure 8 is a bar chart showing *in vitro* permeability of Arg-vasopressin (desmopressin) by itself, in combination with a bile salt, in combination with effervescent composition, and in combination with both bile salt and effervescent composition according to some embodiments of the invention. Fluorescently-labeled desmopressin is measured by fluorescence detection.

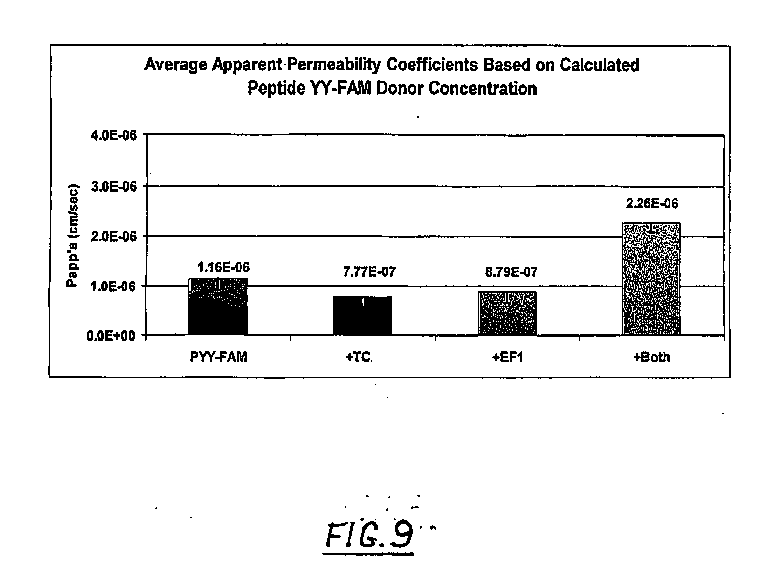

Figure 9 is a bar chart showing *in vitro* permeability of PYY by itself, in combination with a bile salt, in combination with effervescent composition, and in combination with both bile salt and effervescent composition according to some embodiments of the invention. Fluorescently-labeled PYY is measured by fluorescence detection.

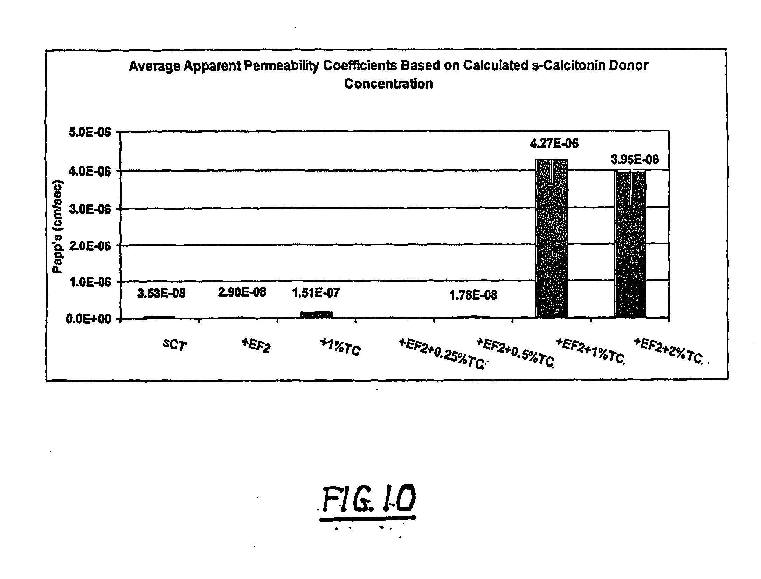

Figure 10 is a bar chart showing *in vitro* permeability of salmon calcitonin by itself, in combination with a bile salt, in combination with effervescent composition, and in combination with both bile salt and effervescent composition according to some embodiments of the invention. Salmon calcitonin is measured by ELISA kit and technique.

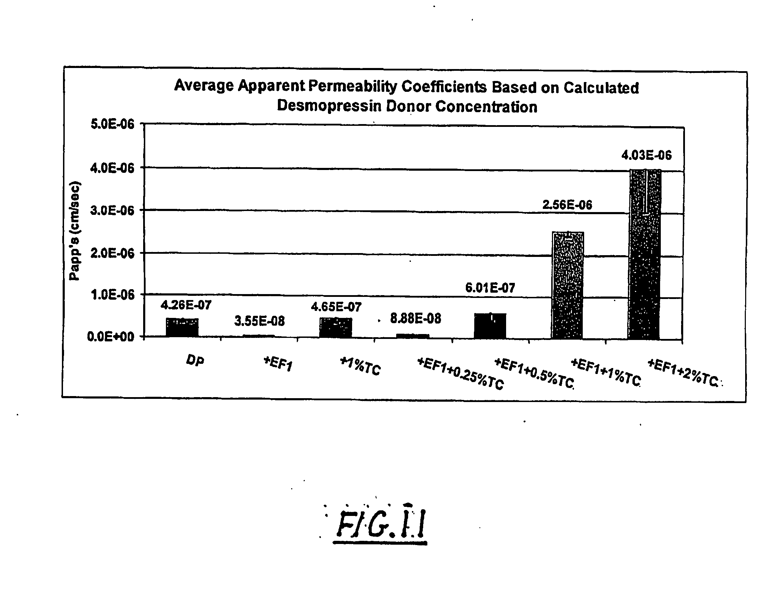

Figure 11 is a bar chart showing *in vitro* permeability of desmopressin (Arg-vasopressin) by itself, in combination with a bile salt, in combination with an effervescent composition, and in combination with both bile salt and effervescent composition according to some embodiments of the invention. Desmopressin is measured by LC/MS/MS.

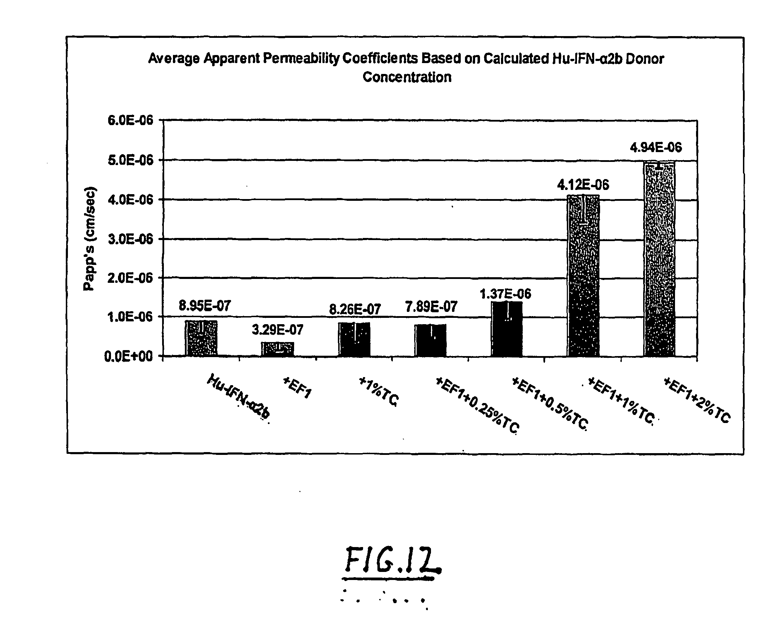

Figure 12 is a bar chart showing *in vitro* permeability of interferon alpha-2b by itself, in combination with a bile salt, in combination with effervescent composition, and in combination with both bile salt and effervescent composition according to some embodiments of the invention. Interferon alpha-2b is measured by ELISA kit and technique.

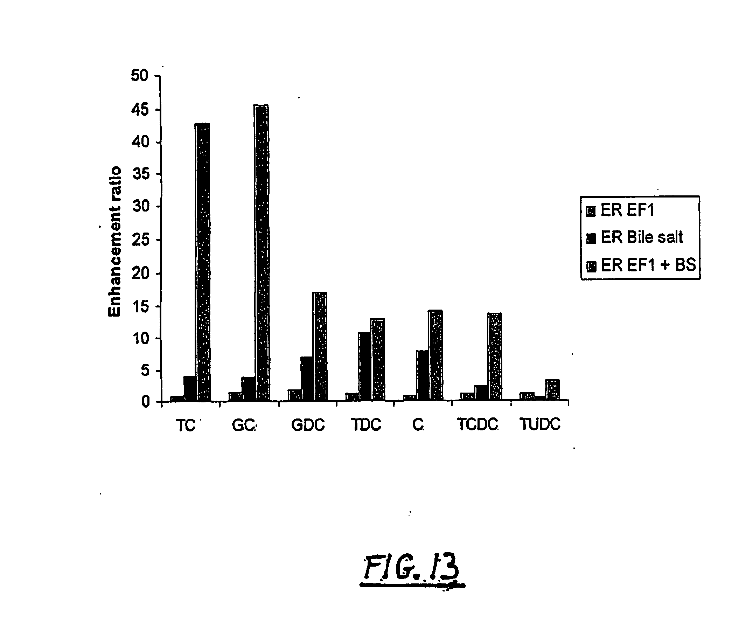

Figure 13 is a bar chart showing the *in vitro* enhancement ratio (ER) of insulin (In-FITC) under different conditions to the permeability of the same concentration of insulin administered alone. The conditions are in combination with bile salt, in combination with effervescent composition, and in combination with both bile salt and effervescent composition according to some embodiments of the invention. Various bile salts tested include (sodium) taurocholate (TC), glycolate (GC), glycodeoxycholate (GDC), taurodeoxycholate (TDC), cholate (C), taurochenodeoxycholate (TCDC), and tauroursodeoxycholate (TUDC). In-FITC is measured by fluorescence detection.

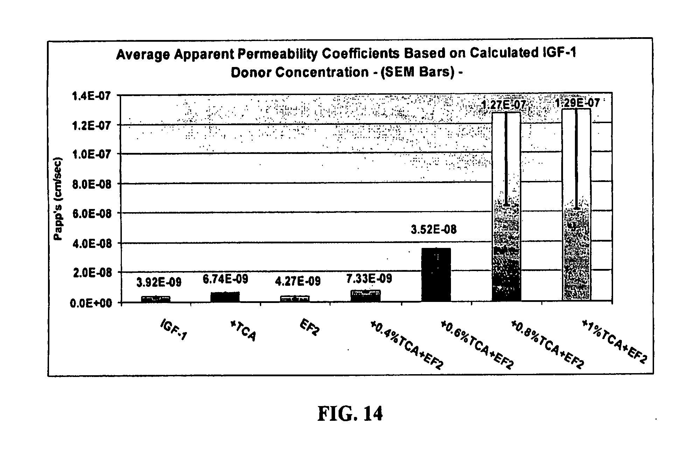

Figure 14 is a bar chart showing *in vitro* permeability of IGF-1 by itself, in combination with a bile salt, in combination with effervescent composition, and in combination with both bile salt (at various concentrations) and effervescent composition. IGF-1 is measured by ELISA kit and technique.

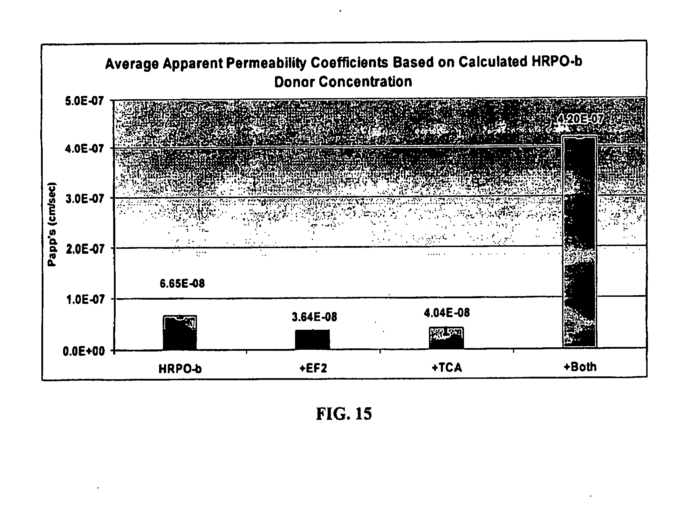

**Figure 15** is a bar chart showing *in vitro* permeability of HRPO-b by itself, in combination with a bile salt, in combination with effervescent composition, and in combination with both bile salt and effervescent composition. HRPO-b is measured by increased optical density due to the conversion of specific substrate by HRPO-b.

## DETAILED DESCRIPTION OF THE INVENTION

As used herein, the phrase "oral transmucosal," within the context of drug delivery and absorption, is meant to refer to the pre-peristaltic stage of uptake of the drug via one or more of the mucosal tissue types associated with the oral cavity, *e.g.*, sublingual, buccal, gingival, palatal, esophageal regions of oromucosal tissue. More specifically, what is intended by the phrase is that the primary delivery route of the active ingredient occurs through the mucosal tissue of the oral cavity. The broader term "transmucosal" is meant to refer to delivery and absorption as accomplished through mucosal tissue, encompassing the mucosal tissue of the mouth, rectum or vagina.

As used herein, the term "about" refers to a range of values from $\pm$ 10% of a specified value, and functional equivalents thereof unless otherwise specifically precluded. For example, the phrase "about 50 mg" includes $\pm$ 10% of 50, or from 45 mg to 55 mg.

As used herein, the phrase "biologically active polypeptide" is meant to refer to polypeptides, peptides and proteins, and refers to a polymeric form of amino acids of any length, which can include coded and non-coded amino acids, chemically or biochemically modified or derivatized amino acids, and polypeptides having modified peptide backbones. The term includes fusion proteins, including, but not limited to, fusion proteins with a heterologous amino acid sequence, fusions with heterologous and homologous leader sequences, with or without N-terminal methionine residues; immunologically tagged proteins; and the like. Biologically active polypeptides useful in the present invention include, without limitation, cytokines, growth factors, hematopoietic factors, hormones, enzymes, antibodies, antigens, allergens, and the like. Biologically active polypeptides share the biological activity associated with the base polypeptide. Biologically active polypeptides can be any length, *e.g.*, from about 5 to about 20 amino acids; from about 10 to about 60 amino acids; from about 25 to about 75 amino acids; from about 50 to about 150 amino acids; from about 75 to about 250 amino acids; from about 100 to about 400 amino acids; from about 200 to about 600 amino acids, and larger.

As used herein, the term "therapeutically effective amount" is meant to refer to the amount determined to be required to produce the physiological effect intended and associated with the given active ingredient, as measured according to established pharmacokinetic methods and techniques, for the given administration route.

As used herein, the phrase "oral dosage form", when used in the general sense, includes orally disintegrable/ dissolvable tablets, capsules, caplets, gels, creams, films, sprays, and the like. The oral dosage form of the invention is meant to include the pharmaceutical composition of the invention as a solid oral dosage form comprising a biologically active polypeptide, accompanied by an excipient formulation which facilitates and enhances oral transmucosal absorption of the active ingredient.

As used herein, the term "substantially", unless otherwise defined, is meant to refer to a specific property, characteristic or variable that meets the stated criteria in such measure that one skilled in the art would understand that the benefit to be achieved, or the condition or property desired, is met.

In general, a pharmaceutical composition according to the invention comprises an effervescent composition in combination with a bile salt, and includes a biologically active polypeptide as the active ingredient. The composition of the invention can further comprise a pH adjusting substance. In some embodiments, the combination of ingredients, *i.e.*, the active ingredient and the excipients used in the dosage form, collectively function to enhance transmucosal absorption of the active ingredient, *e.g.*, the ratio of $C_{max}$ to dosage, and/or dosage reduction realized, in accordance with the invention. In some embodiments, the use of an effervescent composition and a pH adjusting substance, as part of an effervescent excipient component, in conjunction with a bile salt such as sodium taurocholate, provides significant advantages for delivery and/or uptake of polypeptides. In some embodiments, an effervescent composition and pH adjusting substances are used together to increase delivery and/or uptake of polypeptides.

The composition of the invention can be prepared and/or administered in powder form or as a solid oral dosage form, *e.g.* a tablet. It is also possible to deliver the composition of the invention in liquid form. Irrespective of its form, the composition can be administered and/or delivered to a specific oral cavity site wherein the composition dissolves at the oral mucosa site as it comes into contact with the saliva. In some embodiments, the composition dissolves within a period of from about 1 minute to about 30 minutes. The active ingredient is transported across or traverses the oral mucosa in at least the area in which the composition was administered. In some embodiments, the composition comprises an effervescent excipient formulation, a bile salt, and a pharmaceutically active polypeptide, and can be administered and/or delivered to the sublingual, buccal, gingival, palatal, and/or esophageal sites of oromucosal tissue.

In some embodiments, the biologically active polypeptides are transported across the mucosal tissue and absorbed to effectuate their biological effect.

EP 2 155 229 B1

A wide variety of direct and indirect biological activities associated with the various polypeptides can be utilized for a wide range of various treatments or therapies; such therapies include, but are not limited to, antibiotic therapies, hematopoietic therapies, antiallergic therapies, hormone therapies, polypeptide supplement therapies, diagnostic assays, antidepressants and psychotropic therapies, antitumor therapies, antiarrhythmic therapies, vasodilator therapies, vasoconstrictor therapies, antihypertensive therapies, diabetes prevention and control, anticoagulant therapies, appetite suppressant activities, blood glucose control, glycemic control, satiety, anti-infective therapies, osteoporotic therapies, vaccines, and the like. These treatments or therapies could be accomplished upon successful transport of the appropriate polypeptide across the mucosa and into the bloodstream of the recipient.

Suitable biologically active polypeptides include, but are not limited to, amylin, salmon-derived calcitonin (s-CT), glucagon-like peptide 1 (GLP-1), glucagon, parthyroid hormone (PTH1), oxytocin, desmopressin (8 D-Arg vasopressin), insulin, protein YY (PYY), cytokines and lymphokines such as IFNα, IFNβ, IFNγ, and the like. A listing of various polypeptides can be found in Igari et al., U.S. Patent No. 5,725,852.

Biologically active polypeptides that can be used with the invention include those that can transport across or permeate oral mucosal tissue and enter the blood stream to deliver their associated effects. In some embodiments the polypeptides have a molecular weight ranging from about 500 Daltons to about 200,000 Daltons (200 kDa), or greater. In some embodiments the polypeptides have a molecular weight ranging from about 1000 Daltons to about 20,000 Daltons. In some embodiments, the polypeptides have a molecular weight ranging from about 3000 Daltons to about 30,000 Daltons; in other embodiments, the polypeptides have a molecular weight ranging from about 5000 Daltons to 60,000 Daltons.

In some embodiments, the present application further provides methods and compositions for transmucosal delivery of large non-peptide based compounds and molecules, as well as polypeptide-drug conjugates.

The compositions of the invention include at least one bile salt. This portion of the composition is also hereinafter referred to as the "bile salt component" of the composition. "Bile salts" as used herein refer to the cationic salt form of its corresponding bile acid, e.g., bile acid taurocholic acid is (sodium) taurocholate as a bile salt. Bile salts that can be used with the invention include, but are not limited to, bile salts selected from the group consisting of: sodium taurocholate (TC), sodium glycocholate (GC), sodium glycodeoxycholate (GDC), sodium taurodeoxycholate (TDC), sodium cholate (C), sodium taurochenodeoxycholate (TCDC), and sodium tauroursodeoxycholate (TUDC), and combinations thereof. In some embodiments, the bile salt the sodium salt of taurocholic acid, i.e., sodium taurocholate is used. Although for purposes of illustrating the invention sodium is the named cation, it is possible to use other cations to form bile salts.

The amount of bile salt that can be used will vary according to the particular bile salt selected. In some embodiments the amount of bile salt will be relatively low, and within a range of from a minimum effective concentration to achieve the benefits of the invention, and an amount corresponding to maximum acceptable toxicity. The minimum and maximum bile salt amount parameters will differ among the various bile salts. For sodium taurocholate, the amount that can be used for the invention can range from about 0.05% weight to volume to about 10% weight to volume, or between about 0.5% and about 2.0% weight to volume. In some embodiments, about 1.0% weight to volume sodium taurocholate is used.

Pharmaceutical compositions prepared according to the invention comprise an effervescent composition as a penetration enhancer. This portion of the composition of the invention can also be referred to herein as the "effervescent excipient component". In some embodiments, the effervescent excipient component comprises an effervescent couple. An effervescent couple can be an acid and a base that are water or saliva activated; thus, when exposed to water or saliva, e.g., upon dissolution in the mouth, the activation of the effervescent couple results in the production of carbon dioxide. In some embodiments, the effervescent excipient component includes a pH adjusting substance in addition to an effervescent couple. A variety of effervescent compositions or effervescent excipient components can be used in the invention. For example, the effervescent compositions and effervescent excipient components described in U.S. Patent No. 5,178,878 and U.S. Patent No. 5,503,846 can be used.

In some embodiments, effervescent excipient components include effervescent couples that are water- or saliva-activated materials usually kept in anhydrous state with little or no absorbed moisture, or in a stable hydrated form. In some embodiments effervescent couples comprise at least one food grade acid and at least one food grade reactive base, which can be a carbonate or bicarbonate.

Suitable acids for use in the effervescent excipient composition include food grade acids, acid anhydrides and acid salts. Food grade acids include, but are not limited to, citric acid, tartaric acid, malic acid, fumaric acid, adipic acid, ascorbic acid and succinic acid, and acid anhydrides or salts thereof. Salts used can be food grade sodium, potassium and calcium salts, e.g., sodium dihydrogen phosphate and disodium hydrogen phosphate, and acid citrate salts and disodium acid sulfate.

Bases that can be used in accordance with the invention include, but are not limited to, sodium bicarbonate, potassium bicarbonate, and the like. Sodium carbonate, potassium carbonate, magnesium carbonate and the like can also be used to the extent they are used as part of the effervescent couple, but can also be used as a pH adjusting substance within the effervescent composition.

In some embodiments the amount of effervescent excipient component is an effective amount and is determined based on properties other than those which would be necessary to achieve disintegration of a tablet in the mouth. In some embodiments, effervescence is used as a basis for enhancing transmission of the active ingredient across mucosal membranes via buccal, sublingual or gingival administration in the oral cavity. Accordingly, in some embodiments, the amount of effervescent excipient component ranges between about 5 to about 85 percent, between about 15 and 60 percent, between about 30 and 45 percent, and between about 35 and 40 percent, based on total formulation weight. In some embodiments the relative proportion of acid and base depends upon the specific ingredients, *e.g.*, whether the acid is mono-, di- or tri-protic, relative molecular weights, etc.

In some embodiments the pH adjusting substance is an ingredient in addition to and other than one of the components of the effervescent couple. A polypeptide that is susceptible to changes in ionization state can be administered by effecting the proper conditions for its dissolution and transmission across tissues within the oral cavity. In some embodiments, if the ideal conditions for a particular drug are basic, the addition of sufficient excess of a suitable strong acid as part of either the effervescent excipient component or the pH adjusting substance may not be indicated. In some embodiments, a pH adjusting substance, for example anhydrous sodium carbonate, is selected which functions separate and apart from the effervescent couple.

Various pH adjusting substances can be used to provide further permeation enhancement of the active ingredient. In some embodiments, the selection of the appropriate pH adjusting substance depends on the drug to be administered and, in particular, to the pH at which the drug is ionized or unionized, and whether the ionized form or unionized form facilitates transmission across the mucosa.

In some embodiments, the pH adjusting substance is any substance that is capable of adjusting the localized pH to promote transport across the mucosa in amounts which will result in a pH generally ranging from about 3 to about 10, or between about 4 to about 9. The pH is the "localized pH" at the microenvironment at the surface contact area of the oral mucosa and the dosage form (or portions of it as it disintegrates/dissolves) once placed in the mouth of the recipient.

In some embodiments, the localized pH can be determined by initially characterizing the dynamic pH changes displayed by the tablets using *in vitro* pH measurement. The method consists of using 0.5 -10 ml phosphate buffered saline in an appropriately sized test tube or other similar vessel. One liter volume of buffered saline solution can be prepared by dissolving 9.0 g sodium chloride, 0.6 g sodium phosphate monobasic monohydrate and 0.78 g of sodium phosphate dibasic (anhydrous) in about 1000 ml of deionized water, and adjusting the pH to 7.0 $\pm$ 0.05 at room temperature by adding 1 N sodium hydroxide with stirring. The adjustment should require about 0.5 ml. The amount of media used depends on the tablet size and dosage. For example, a volume of 1 ml can be used for a tablet weighing 200 mg. Immediately upon contact with the media, the pH profile of the solution is monitored as a function of time, using a micro-combination pH electrode.

In some embodiments, the materials which can be used as pH adjusting substances in accordance with the present invention include carbonate, bicarbonate, phosphate, hydrogen phosphate and dihydrogen phosphate. Suitable carbonates include, without limitation, sodium carbonate, potassium carbonate or calcium carbonate. Suitable phosphates include, without limitation, calcium phosphate or sodium phosphate. In some embodiments, the pH adjusting substance is sodium carbonate. In some embodiments, the pH adjusting substances, when provided in suitable amount, provide a change in localized pH of at least about 0.5 pH units, 1.0 pH units, or about 2.0 pH units when compared to an otherwise identical formulation without the pH adjusting substance.

In some embodiments, the amount of pH adjusting substance varies with the type of pH adjusting substance used, amount of excess acid or base from the effervescent couple, the nature of any remaining ingredients, and the active ingredient. In some embodiments, the amount of pH adjusting substance varies from about 0.5 to about 25 percent, between about 2 to about 20 percent, between about 5 to about 15 percent, and between about 7 and about 12 percent by weight of the total formulation weight.

When the composition is in the solid dosage form of a tablet, in some embodiments the composition further comprises one or more of a filler, disintegrant, and lubricant. Any filler or any amount of a filler can be used as long as the resulting dosage forms achieve the results described herein. In some embodiments, the fillers are sugar and sugar alcohols, and these may include non-direct compression and direct compression fillers. In some embodiments non-direct compression fillers ,when formulated, have flow and/or compression characteristics which make them impractical for use in high speed tableting process without augmentation or adjustment. For example, a formulation may not flow sufficiently well and therefore, a glidant such as silicon dioxide may need to be added.

In some embodiments, direct compression fillers do not require similar allowances and generally have compressibility and flowability characteristics which allow them to be used directly. In some embodiments, non-direct compression fillers may be imparted with the properties of direct compression fillers. In some embodiments, non-direct compression fillers tend to have relatively smaller particle size when compared to direct compression fillers. In some embodiments, fillers such as spray dried mannitol have relatively smaller particle sizes and yet are often directly compressible, depending on how they are further processed. In some embodiments, the fillers are large non-direct compression fillers.

Suitable fillers for use with the invention include, but are not limited to, mannitol, lactose, sorbitol, dextrose, sucrose, xylitol and glucose. In some embodiments, the filler is spray dried mannitol. The amount of filler used can range from about 10 to about 80 percent, from about 25 to about 80 percent, or from about 35 to about 60 percent by weight of the formulation.

Disintegrants can also be used in the composition. In some embodiments, disintegrants can permit dosage reduction and/or increase the ratio of $C_{max}$ and dose. In some embodiments, disintegrants include binders that also have disintegrant properties. Suitable disintegrants include, but are not limited to, microcrystalline cellulose, cross-linked polyvinyl pyrrolidone (PVP-XL), sodium starch glycolate, croscarmellose sodium, cross-linked hydroxypropyl cellulose, and the like. In some embodiments, selection of the disintegrant can depend upon whether or not, within a given system, the results described can be obtained with its use.

In some embodiments, the disintegrant is a starch glycolate. In some embodiments, the disintegrant is sodium starch glycolate. An example of a sodium starch glycolate is EXPLOTAB™ (standard grade, available from Roquette of Lestrem, France).

In some embodiments, the amount of disintegrant varies according to factors such as dosage form size, nature and amount of other ingredients, and the like. In some embodiments the amount of disintegrant ranges from about 0.25% to about 20% by weight of the final formulation, between about 0.5% and about 15% w/w, between about 0.5% and about 10% w/w, or between about 1% and about 8% by weight, based on the weight of the finished formulation.

In some embodiments, the invention further comprises a tableting or ejection lubricant. Suitable lubricants include, but are not limited to, magnesium stearate, stearic acid, calcium stearate, and combinations thereof. In some embodiments the lubricant is magnesium stearate. In some embodiments, the amount of lubricant is less than 1% of the formulation by weight. In some embodiments, the amount of lubricant is less than about 0.5%. In some embodiments, the amount of the lubricant can be greater than about 1.0%, greater than 1.5% and between about 1.5% and about 3%. In some embodiments, magnesium stearate is the lubricant and is used at about 2% by weight.

In some embodiments, the composition of the invention includes other conventional excipients in generally known amounts, provided they do not significantly detract from the advantageous attributes afforded by the invention. Such additional excipients can include, but are not limited to, binders, sweeteners, coloring agents, flavoring agents, glidants, lubricants, disintegrants, preservatives, and the like.

In some embodiments, the composition of the invention can be prepared as a solid oral transmucosal dosage form, *e.g.*, a tablet. Effervescent tablets prepared in accordance with the invention can be relatively robust or soft. For example, tablets containing the composition of the invention can be prepared according to the methods described in U.S. Patent No. 5,178,878. When prepared according to this technique, the dosage form can have a hardness of less than about 15 Newtons. The active ingredient may be coated with a protective material or may be uncoated. When soft friable tablets are produced, they can be packaged in blister packs such as those described in U.S. Patent No. 6,155,423. In some embodiments robust dosage forms with a hardness of greater than about 15 Newtons can be manufactured according to the process described in U.S. Patent No. 6,024,981. Further, the degree of state of powder, *e.g.*, reproducibility and/or consistency of particle size, can affect results.

The overall dosage form dimensions and size, and the dosage amount of active ingredient, can vary. The overall shape or configuration of the dosage form, such as tablet, can vary. Dosage form configuration can differ depending upon the intended locale for its residence during administration, *e.g.*, buccal, gingival, or sublingual. In some embodiments, the *in situ* disintegration or dissolution time (dwell time) achieved by the invention is a period sufficient to deliver a therapeutically effective amount of the pharmaceutically active polypeptide across the mucosa. This period can be less than about 30 minutes, or even less than about 20 minutes. In some embodiments, dwell time can range from about 5 minutes to 10 minutes, depending upon the patient response and composition ingredients.

Tablet dosage forms can be prepared using conventional tableting equipment and techniques readily available to those skilled in the art. In some embodiments, tablets made in accordance with the invention are dry blended and directly compressed. In some embodiments, tablets can be prepared from granulation. Dry granulation techniques can be used. For example, granulated mannitol can be used as a filler. As part of the preparation process, in some embodiments, it may be desirable to granulate or pre-mix a portion of the composition prior to final blending and compression. Materials selected are pre-selected to provide the intended dose and content uniformity. Thus, in some embodiments, an appropriate amount of effervescent couple, pH adjusting substance and disintegrant can be selected, and provided in predetermined amounts and formulated into the desired dosage form. When lubricants such as magnesium stearate are used, in some embodiments, they are added toward the end of the blending period, *e.g.*, a few minutes prior to final cessation of blending.

In some aspects, the invention also includes methods of administering a biologically active polypeptide to a recipient comprising:

a) providing an oral transmucosal composition of a pharmaceutically active polypeptide, effervescent composition and bile salt;

b) placing the oral transmucosal composition at a mucosal tissue site within the oral cavity of the recipient; and

c) permitting the composition to dissolve *in situ* for a period sufficient to permit dissolution of the dosage form and to deliver a therapeutically effective amount of the polypeptide across the mucosa.

The term "providing" is meant to include removal of the composition or its dosage form from packaging or containment, and/or having another dispense the dosage form. As discussed above, dosage forms prepared in accordance with the invention can be presented in blister packages to the recipient. As used herein, the term "recipient" is meant to be inclusive of mammals, including humans. The recipient, or another individual, can place the composition between the cheek and upper or lower gum, or sublingually. In some embodiments, the composition is provided with instructions that the composition should not be sucked, chewed or swallowed.

**Therapeutic uses**

A number of therapeutic uses can be employed with the oral transmucosal compositions of the present disclosure, including therapeutic uses for diabetes control. The invention provides an oral transmucosal composition comprising therapeutically effective amount of insulin, an effervescent excipient component; and a bile salt for use in methods of treating diabetes in a recipient.

The invention is further illustrated by the following examples, none of which are to be construed as necessarily limiting the invention.

**EXAMPLES**

**Example 1**

***In Vitro* Permeability Assay**

*In vilro* permeability testing was conducted as follows. Starting with acrylic inserts having one end sealed with 0.6 cm square area inserts of acrylic and polycarbonate membranes without cells, the cultured human buccal carcinoma cells (SqCC/Y1 cells) were grown on the membrane surface in culture medium for a period of 24 hours. The culture medium was removed from the top of the insert ("air-lifting" process), wherein the cells continued to receive nutrients from the culture medium beneath the inserts. Under these conditions, the cells were grown for a period of 7 to 10 days so as to grow the cells to multilayer confluence on the polycarbonate membrane, thereby increasing cellular density to a multi-layer, tissue-like barrier resembling that of buccal mucosal tissue.

The assay was performed by rinsing the inserts with Krebs-Ringer buffer solution without sodium bicarbonate (KRB) and inspecting for uniformity by measuring the transepithelial electrical resistance (TEER). When the average electrical resistance necessary was 350 ohms or greater, the inserts were selected for permeability testing, thereby ensuring consistent permeability barrier thickness and distribution.

The inserts were separated into the groups (e.g., n=3) with closely similar average TEER values (of the group) to be used in testing particular formulations of active pharmaceutical ingredients (i.e., polypeptide). For a given bile salt, for example, the sample groups were as follows:

1) polypeptide alone;

2) polypeptide in combination with bile salt;

3) polypeptide in combination with effervescent excipient composition (Excipient Formula 1) powder;

4) polypeptide in combination with bile salt (varying concentrations) and effervescent excipient composition (Excipient Formula 1) powder.

The inserts were placed into cell culture wells with a defined volume of KRB and incubated at 37° C on shaker tables set at 100 rpm. Thus, the cells were exposed to the given active ingredient (polypeptide) without and in combination with the various excipient formulations described.

Samples were removed from the basolateral fluid every 10 minutes for a period of one hour, and the removed fluid was replaced with an equal volume of KRB. At the end of the hour, the residual fluid within the insert was recovered and stored, the insert was rinsed with KRB, and the TEER value again measured. The TEER differences between the starting step TEER and final step TEER were recorded.

The inserts were then evaluated for cytotoxicity of the treatment using the MTS toxicity assay, and the effects of the treatments in the samples were also recorded. The MTS toxicity assay measures the ability of live cells to convert the MTS tetrazolium compound into formazan, which absorbs UV light at 490 nm. The amount of absorbance is directly proportional to the number of viable cells.

The samples from the basolateral wells were analyzed for the active ingredient (polypeptide) using various techniques. The amount of active in the well was calculated and corrected for sampling loss. From this data, the amount of active ingredient permeating the cell barrier was calculated and the Apparent Permeability Coefficient (Papp) was calculated:

$$Papp = S/(A*C)$$

wherein

S = slope of permeability curve in the linear region (mg/sec);

A = area of the insert covered with cells (cm$^2$);

C = concentration of donor active pharmaceutical ingredient (mg/ml).

Enhancement Ratio (ER) is equal to the ratio of the Papp with absorption enhancer divided by the Papp without absorption enhancer. Thus, this technique was used for each of the proposed permeability enhancement compositions below.

**Example 1A** Preparation of Excipient Formulations

**Excipient Formula 1 (EF 1)**

The first excipient component comprises preparing the effervescent composition in dry powder form. In some embodiments, the effervescent composition that is used is the excipient formulation powder set forth in the following table:

Table 1 Effervescent Excipient Component 200 mg Placebo Tablet

| Ingredient: | Amount (mg) | Amount % weight |
|---|---|---|
| Mannitol EZ | 98 | 49% |
| Sodium bicarbonate | 42 | 21% |
| Citric acid | 30 | 15% |
| Sodium carbonate | 20 | 10% |
| Sodium starch glycolate | 6 | 3% |
| Magnesium stearate | 4 | 2% |
| **Total:** | 200 mg | 100% |

**Excipient Formula 2 (EF 2)**

An alternative effervescent composition was prepared based on Excipient Formula 1, except using only the effervescent couple and a pH adjusting substance. This formulation was prepared to the same capacity as 200 mg of Excipient Formula 1:

Table 2 Effervescent Excipient Component 92 mg Placebo Powder

| Ingredient: | Amount (mg) | Amount % weight |
|---|---|---|
| Sodium bicarbonate | 42 | 45.7% |
| Citric acid | 30 | 32.6% |
| Sodium carbonate | 20 | 21.7% |
| **Total:** | **92 mg** | **100%** |

**Example 1B** Active Ingredient and Bile Salt Component

The second active component comprises the bile salt sodium taurocholate in combination with the biologically active polypeptide as prepared in solution form. For example, the dry-form sodium taurocholate and polypeptide ingredient can be reconstituted into a buffered saline solution. One buffered saline solution can be composed of, for example, Krebs Ringer Buffer (D-glucose 1.8 g/L, magnesium chloride (anhydrous) 0.0468 g/L, potassium chloride 0.34 g/L, sodium chloride 7.0 g/L, sodium phosphate dibasic (anhydrous) 0.1 g/L, sodium phosphate monobasic (anhydrous) 0.18 g/L; pH adjusted to 7.4; no sodium bicarbonate used.

Thus, in some embodiments, the compositions of the invention comprise the combination of the above components - effervescent excipient component in powder form, and active component containing the bile salt and biologically active polypeptide in solution - deposited in sequence. The two-part component composition can be deposited in sequence, the powder composition followed by the liquid composition, onto the mucosal tissue.

**Example 1C** Preparation of Solid Oral Transmucosal Dosage Form Containing Polypeptides

Alternatively to the composition described in Example 1A and 1B, in some embodiments the compositions of the invention can be prepared as a solid oral transmucosal dosage form, *e.g.*, compressed tablet. The tablet dosage form can be prepared by mixing the effervescent composition or effervescent excipient composition, bile salt, and biologically active polypeptide in powder form, followed by compressing the powder mixture in a tablet press to form the resulting solid tablet. In some embodiments, solid dosage forms in the form of a tablet can be prepared using conventional techniques and equipment readily available to those skilled in the art.

### *In Vitro* Testing using Fluorescent-Labeled Polypeptides

When the *in vitro* permeability assay is used, the polypeptides used are often those conjugated to a fluorescent moiety. In particular, the fluorescent moiety used was selected from FAM (5(6) carboxyfluorescein) and FITC (fluorescein isothiocyante). Fluorescent-conjugated polypeptides permit the use of a single sensitive assay technique for any given polypeptide that includes the same moiety, regardless of polypeptide molecular weight. This, in turn, enables the evaluation of a large variety and range of polypeptides using a single assay without the need to develop specific assays for each particular polypeptide.

The permeability of fluorescently-labeled polypeptide with and without enhancement was measured by the *in vitro* permeability assay. The amount of polypeptide permeating through the cells/tissue into the basolateral fluid was measured and the apparent permeability coefficient (Papp) for each polypeptide sample was calculated. The Papp of the combination of bile salt (taurocholate) and the effervescent powder was compared to the Papp values obtained with the polypeptide alone, the polypeptide in combination with effervescent composition without bile salt, and the polypeptide with bile salt (sodium taurocholate) without the effervescent composition to determine if there was synergy between the two enhancer components when combined. When the taurocholate alone was used in the testing, the taurocholate powder was not deposited directly onto the cell surface to protect from toxic effect on cells.

**Example 2** Comparative *In Vitro* Permeability of Amylin

*In vitro* permeability was evaluated for the polypeptide amylin. Amylin is associated with glycemic control and treatment of diabetes. FAM-labeled amylin is a 37 amino acid peptide having a MW of 4259.86 Daltons. Using the cell culture model described above, the permeability of amylin-FAM was tested for the following formulations:

Table 3 Comparative Amylin-FAM Formulations

| Ingredients in KRB: | Am only | Am + EF 1 | Am + TC | Am + EF 1 + TC |
|---|---|---|---|---|
| Amylin-FAM | 100 pM/ml | 100 pM/ml | 100 pM/rol | 100 pM/ml |
| EF 1 | - | 40 mg/ml | - | 40 mg/ml |
| Na Taurocholate | - | - | 10 mg/ml | 10 mg/ml |
| Am = amylin; EF1= Excipient Formula 1; TC = sodium taurocholate. KRB = Krebs Ringer Buffer solution. | | | | |

The results are shown in Figure 1. As can be seen from the data in the chart, amylin as the active polypeptide together with the bile salt alone at the given concentration does not appear to appreciably increase the absorption of the polypeptide across mucosal tissue in the *in vitro* model. Likewise, the effervescent excipient component (Excipient Formula 1 described above in Example 1A) alone with the polypeptide amylin-FAM also does not appear to appreciably enhance the absorption across mucosal tissue.

The combination of effervescence with the 1% bile salt, however, appears to enhance the transport/permeability of the polypeptide across the mucosal tissue. When used together, the combination of taurocholate and effervescent excipient component with the polypeptide produced a permeation result greater than the sum of the individual permeation effects of bile salt and effervescent component by themselves.

**Example 3** Comparative *In Vitro* Permeability of s-CT

*In vitro* permeability was evaluated for the polypeptide salmon-derived calcitonin (s-CT) associated with calcium metabolism and the treatment of osteoporosis. IT has also been reported to be a satiety hormone. FAM-labeled s-CT is a 25 amino acid peptide fragment (8-32) having a molecular weight of 3441.1 Daltons. Using the cell culture model described above, the permeability of s-CT-FAM was tested for the following formulations:

Table 4 Comparative s-CT-FAM Formulations

| Ingredients (in KRB): | s-CT only | s-CT + EF 1 | s-CT + TC | s-CT + EF 1 + TC |
|---|---|---|---|---|
| s-CT-FAM | 100 pM/ml | 100 pM/ml | 100 pM/ml | 100 pM/ml |
| EF 1 | - | 40 mg/ml | - | 40 mg/ml |
| Na Taurocholate | - | - | 10 mg/ml | 10 mg/ml |
| s-CT = salmon-derived calcitonin; EF1= Excipient Formula 1; TC = sodium taurocholate. KRB = Krebs Ringer Buffer solution. | | | | |

The results are shown in Figure 2. As can be seen from the data in the chart, The s-CT-FAM as the active polypeptide together with the bile salt alone at the given concentration does not appear to appreciably increase the permeability of the polypeptide across mucosal tissue in the *in vitro* model. Likewise, the effervescent excipient component Excipient Formulation 1 described above in Example 1A) alone with the polypeptide s-CT-FAM also does not appear to appreciably enhance the absorption across mucosal tissue.

The combination of effervescence with the 1% bile salt, however, appears to enhance the transport/permeability of the polypeptide across the mucosal tissue. When used together, the combination of taurocholate and effervescent excipient component with the polypeptide appears to produce a permeation result greater than the sum of the individual permeation effects of bile salt and effervescent component by themselves.

**Example 4** Comparative *In Vitro* Permeability of GLP-1-FAM

*In vitro* permeability was evaluated for glucagon-like peptide 1, also known as GLP-1. GLP-1 is associated with treating type 2 diabetes, as well as satiety control and weight loss. GLP-1-FAM is a 30 amino acid peptide having a molecular weight of 4071.71. Using the cell culture model described above, the permeability of GLP-1-FAM was tested for the following formulations:

Table 5 Comparative GLP-1-FAM Formulations

| Ingredients (in KRB): | GLP1 only | GLP1 + EF 1 | GLP1 + TC | GLP1 + EF 1 + TC |
|---|---|---|---|---|
| GLP-1-FAM | 100 pM/ml | 100 pM/ml | 100 pM/ml | 100 pM/ml |
| EF 1 | - | 40 mg/ml | - | 40 mg/ml |
| Na Taurocholate | - | - | 10 mg/ml | 10 mg/ml |
| GLP1 = GLP-1; EF1= Excipient Formula 1; TC = sodium taurocholate. KRB = Krebs Ringer Buffer solution. | | | | |

The results are shown in Figure 3. As can be seen from the data in the chart, The GLP-1-FAM as the active polypeptide together with the bile salt alone at the given concentration does not appear to appreciably increase the permeability of the polypeptide across mucosal tissue in the *in vitro* model. Likewise, the effervescent excipient component (Excipient Formula 1 described above in Example 1A) alone with the polypeptide GLP-1-FAM also does not appear to appreciably enhance the absorption across mucosal tissue.

The combination of effervescence with the 1% bile salt, however, does appears to enhance the transport/ permeability of the polypeptide across the mucosal tissue. When used together, the combination of taurocholate and effervescent excipient component with the polypeptide appear to produce a permeation result greater than the sum of the individual permeation effects of bile salt and effervescent component by themselves.

**Example 5** Comparative *In Vitro* Permeability of Insulin-FITC

*In vitro* permeability was evaluated for insulin. Insulin is a 51 amino acid polypeptide having a molecular weight of 5808 Daltons and associated with treatment of diabetes. The insulin used for the test was conjugated to the fluorescent moiety FITC (fluorescein isothiocyanate). Using the cell culture model described above, the permeability of insulin-FITC was tested for the following formulations:

Table 6 Comparative Insulin-FITC Formulations

| Ingredients (in KRB): | In only | In + EF 1 | In + TC | In + EF 1 + TC |
|---|---|---|---|---|
| In-FITC | 50 pM/ml | 50 pM/ml | 50 pM/ml | 50 pM/ml |
| EF 1 | - | 40 mg/ml | - | 40 mg/ml |
| Na Taurocholate | - | - | 10 mg/ml | 10 mg/ml |
| In = insulin; EF1= Excipient Formula 1; TC = sodium taurocholate. KRB = Krebs Ringer Buffer solution. | | | | |

The results are shown in Figure 4. As can be seen from the data in the chart, insulin-FITC as the active polypeptide together with the bile salt alone at the given concentration does not appear to appreciably increase the permeability of the polypeptide across mucosal tissue in the *in vitro* model. Likewise, the effervescent excipient component (Excipient Formula 1 described above in Example 1A) alone with the polypeptide insulin also does not appear to enhance the absorption across mucosal tissue.

The combination of effervescence with the 1% bile salt, however, appears to enhance the transport/permeability of the polypeptide across the mucosal tissue. When used together, the combination of taurocholate and effervescent excipient component with the polypeptide appears to produce a permeation result greater than the sum of the individual permeation effects of bile salt and effervescent component by themselves.

**Example 6** Comparative *In Vitro* Permeability of Glucagon-FAM

*In vitro* permeability was evaluated for glucagon, which is associated with treatment of acute hypoglycemia. Glucagon-FAM is an 11 amino acid peptide having a molecular weight of 1709.63 Daltons. Using the cell culture model described above, the permeability was tested for the following formulations:

Table 7 Comparative Glucagon-FAM Formulations

| Ingredients (in KRB): | Gluc only | Gluc + EF 1 | Gluc + TC | Gluc + EF 1 + TC |
|---|---|---|---|---|
| Gluc-FAM | 100 pM/ml | 100 pM/ml | 100 pM/ml | 100 pM/ml |
| EF 1 | - | 40 mg/ml | - | 40 mg/ml |
| Na Taurocholate | - | - | 10 mg/ml | 10 mg/ml |
| Gluc = glucagon; EF1= Excipient Formula 1; TC = sodium taurocholate. KRB = Krebs Ringer Buffer solution. | | | | |

The results are shown in Figure 5. As can be seen from the data in the chart, glucagons-FAM as the active polypeptide together with the bile salt alone at the given concentration does not appear to appreciably increase the permeability of the polypeptide across mucosal tissue in the *in vitro* model. Likewise, the effervescent excipient component (Excipient Formula 1 described above in Example 1 A) alone with the polypeptide glucagon-FAM also does not appear to appreciably enhance the absorption across mucosal tissue.

The combination of effervescence with the 1% bile salt, however, does appear to enhance the transport/ permeability of the polypeptide across the mucosal tissue. When used together, the combination of taurocholate and effervescent excipient component with the polypeptide produced a permeation result greater than the sum of the individual permeation effects of bile salt and effervescent component by themselves.

**Example 7** Comparative *In Vitro* Permeability of PTH

*In vitro* permeability was evaluated for parathyroid hormone (PTH). PTH is a 38 amino acid peptide associated with the treatment of osteoporosis. PTH-FAM has a molecular weight of 5174.2 Daltons. Using the cell culture model described above, the permeability of PTH-FAM was tested for the following formulations:

**Table 8** Comparative PTH-FAM Formulations

| Ingredients (in KRB): | PTH only | PTH + EF 1 | PTH + TC | PTH + EF 1 + TC |
|---|---|---|---|---|
| PTH-FAM | 100 pM/ml | 100 pM/ml | 100 pM/ml | 100 pM/ml |
| EF 1 | - | 40 mg/ml | - | 40 mg/ml |
| Na Taurocholate | - | - | 10 mg/ml | 10 mg/ml |
| PTH = parathyroid hormone; EF1= Excipient Formula 1; TC = sodium taurocholate. KRB = Krebs Ringer Buffer solution. | | | | |

The results are shown in Figure 6. As can be seen from the data in the chart, PTH-FAM as the active polypeptide together with the bile salt alone at the given concentration does not appear to appreciably increase the permeability of the polypeptide across mucosal tissue in the *in vitro* model. Likewise, the effervescent excipient component (Excipient Formulation 1 described above in Example 1A) alone with the polypeptide PTH-FAM also does not appear to appreciably enhance the absorption across mucosal tissue.

The combination of effervescence with the 1% bile salt, however, does appear to enhance the transport/ permeability of the polypeptide across the mucosal tissue. When used together, the combination of taurocholate and effervescent excipient component with the polypeptide appeared to produce a permeation result greater than the sum of the individual permeation effects of bile salt and effervescent component by themselves.

**Example 8** Comparative *In Vitro* Permeability of Oxytocin-FAM

*In vitro* permeability was evaluated for oxytocin, a hormone associated with uterine contractions. Oxytocin-FAM is a peptide having 9 amino acids and a molecular weight of about 1365.18 Daltons. Using the cell culture model described above, the permeability of oxytocin-FAM was tested for the following formulations:

Table 9 Comparative Oxytocin-FAM Formulations

| Ingredients (in KRB): | Oxy only | Oxy + EF 1 | Oxy + TC | Oxy + EF 1 + TC |
|---|---|---|---|---|
| Oxy-FAM | 100 pM/ml | 100 pM/ml | 100 pM/ml | 100 pM/ml |
| EF 1 | - | 40 mg/ml | - | 40 mg/ml |
| Na Taurocholate | - | - | 10 mg/ml | 10 mg/ml |
| Oxy = oxytocin; EF1= Excipient Formula 1; TC = sodium taurocholate. KRB = Krebs Ringer Buffer solution. | | | | |

The results are shown in Figure 7. As can be seen from the data in the chart, oxytocin-FAM as the active polypeptide together with the bile salt alone at the given concentration does not appear to appreciably increase the permeability of the polypeptide across mucosal tissue in the *in vitro* model. Likewise, the effervescent excipient component (Excipient Formula 1 described above in Example 1A) alone with the polypeptide oxytocin-FAM also does not appear to appreciably enhance the absorption across mucosal tissue.

The combination of effervescence with the 1% bile salt, however, does appear to enhance the transport/ permeability of the polypeptide across the mucosal tissue. When used together, the combination of taurocholate and effervescent excipient component with the polypeptide appear to produce a permeation result greater than the sum of the individual permeation effects of bile salt and effervescent component by themselves.

**Example 9** Comparative *In Vitro* Permeability of Desmopressin-FAM

*In vitro* permeability was evaluated for 8 D-Arg vasopressin (AVP), also known as desmopressin. Desmopressin is associated with vasoconstriction and diuretic therapy treatments. Desmopressin-FAM is a 9 amino acid peptide having a molecular weight of 1442.25 Daltons. Using the cell culture model described above, the permeability of desmopressin-FAM was tested for the following formulations:

Table 10 Comparative AVP-FAM Formulations

| Ingredients (in KRB): | AVP only | AVP + EF 1 | AVP + TC | AVP + EF 1 + TC |
|---|---|---|---|---|
| AVP-FAM | 100 pM/ml | 100 pM/ml | 100 pM/ml | 100 pM/ml |
| EF 1 | - | 40 mg/ml | - | 40 mg/ml |
| Na Taurocholate | - | - | 10 mg/ml | 10 mg/ml |
| AVP = 8-Arg vasopressin; EF1= Excipient Formula 1; TC = sodium taurocholate. KRB = Krebs Ringer Buffer solution. | | | | |

The results are shown in Figure 8. As can be seen from the data in the chart, desmopressin-FAM, or AVP-FAM, as the active polypeptide together with the bile salt alone at the given concentration does not appear to appreciably increase the permeability of the polypeptide across mucosal tissue in the *in vitro* model. Likewise, the effervescent excipient component (Excipient Formula 1 described above in Example 1A) alone with the polypeptide desmopressin-FAM also does not appear to appreciably enhance the absorption across mucosal tissue.

The combination of effervescence with the 1% bile salt, however, does appear to enhance the transport/permeability of the polypeptide across the mucosal tissue. When used together, the combination of taurocholate and effervescent excipient component with the polypeptide appear to produce a permeation result greater than the sum of the individual permeation effects of bile salt and effervescent component by themselves.

**Example 10** Comparative *In Vitro* Permeability of PYY-FAM

*In vitro* permeability was evaluated for PYY, or protein YY. PYY is a 34 amino acid peptide associated with the treatment of obesity. PYY-FAM has a molecular weight of 4407.71. Using the cell culture model described above, the permeability of PYY-FAM was tested for the following formulations:

Table 11 Comparative PYY-FAM Formulations

| Ingredients (in KRB): | PYY only | PYY + EF 1 | PYY + TC | PYY + EF 1 + TC |
|---|---|---|---|---|
| PYY-FAM | 100 pM/ml | 100 pM/ml | 100 pM/ml | 100 pM/ml |
| EF 1 | - | 40 mg/ml | - | 40 mg/ml |
| Na Taurocholate | - | - | 10 mg/ml | 10 mg/ml |
| PYY = peptide YY; EF1= Excipient Formula 1; TC = sodium taurocholate. KRB = Krebs Ringer Buffer solution. | | | | |

The results are shown in Figure 9. As can be seen from the data in the chart, PYY-FAM as the active polypeptide together with the bile salt alone at the given concentration does not appear to appreciably increase the absorption of the polypeptide across mucosal tissue in the *in vitro* model. Likewise, the effervescent excipient component (Excipient Formula I described above in Example 1A) alone with the polypeptide PYY-FAM also does not appear to appreciably enhance the absorption across mucosal tissue.

The combination of effervescence with the 1% bile salt, however, does appear to enhance the transport/permeability of the polypeptide across the mucosal tissue. When used together, the combination of taurocholate and effervescent excipient component with the polypeptide appear to produce a permeation result greater than the sum of the individual permeation effects of bile salt and effervescent component by themselves.

Other methods that did not employ fluorescent labeled polypeptides were also utilized to confirm synergistic permeation enhancement effects of the invention.

**Example 11** s-CT Analysis by ELISA

Salmon calcitonin, or s-CT, was measured by an ultra-sensitive ELISA kit DSL-10-3600 (acquired from Diagnostic Systems Laboratories, Inc., Webster, Texas). Using this technique, s-CT is captured by a plastic-bound antibody and developed using an enzyme-linked specific antibody that provides optical density when incubated with the enzyme-specific substrate. Thus, the amount of s-CT passing through the cultured buccal cells was measured over time and used to calculate apparent permeability coefficients (Papp).

The results are depicted in Figure 10. As can be seen from the data in the figure, s-CT as the active polypeptide combined with the bile salt (sodium taurocholate) alone, or the polypeptide combined with the effervescent composition (effervescent Excipient Formula 2 described in Example 1A and referred to as EF 2) alone, does not appear to exhibit an increase in permeation of the polypeptide s-CT across mucosal tissue in the *in vitro* model.

The combination of the polypeptide s-CT, effervescent formulation and 1% or 2% bile salt, however, does appear to exhibit appreciably enhanced permeation/transport of the polypeptide across the mucosal tissue. When used together, the combination of taurocholate and the effervescent excipient formulation with the polypeptide appear to produce a permeation result greater than the sum of the individual permeation effects of the polypeptide with the same bile salt alone and same effervescent formulation alone.

### Example 12 Desmopressin Analysis by HPLC-MS-MS

A liquid chromatography (LC) - mass spectroscopy (MS) technique was developed to measure the amount of desmopressin passing through the cultured buccal cells over time. This data was then used to calculate the apparent permeability coefficient (Papp). Using the compiled comparative testing information, Figure 11 was prepared.

Desmopressin (8-Arg vasopressin and shown in the figure as DP) as the active polypeptide in combination with the bile salt (sodium taurocholate) at a given concentration, or in combination with the effervescent formulation alone (Excipient Formula 2 described above in Example 1A and a.k.a. EF 2), does not appear to appreciably increase polypeptide permeation across the mucosal tissue in the *in vitro* model. The combination of polypeptide (DP), effervescent formulation and 1% or 2% bile salt, however, appears to exhibit appreciable enhancement of transport/permeability of the polypeptide across the mucosal tissue. Thus, the LC/MS/MS technique for peptide quantification generally support and corroborate the results seen using the fluorescent-labeled polypeptide techniques described herein above.

### Example 13 IFN alpha-2b Analysis by ELISA

Recombinant human interferon alpha-2b (IFN$\alpha$) (molecular weight of 19,271 Daltons and having 166 amino acids, and having a specific activity of 260 million IU per mg protein) (INTRON™ A acquired from Schering Corp., Kenilworth, New Jersey) was tested as the biologically active polypeptide with the invention. IFN$\alpha$ is a cytokine involved with immune function related to viral infections. Active polypeptide was measured using ELISA technique (ELISA kit 41110-2 acquired from PBL Interferon Source, Inc., Piscataway, New Jersey). In this assay, the amount of IFN$\alpha$ passing through the cultured buccal cells was measured over time and used to calculate apparent permeation coefficient (Papp). The data is shown in Figure 12.

As can be seen in the figure, IFN$\alpha$ as the biologically active polypeptide in combination with the bile salt alone and effervescent composition (Excipient Formula 1 as described in Example 1A and referred to as EF 1) alone does not appear to appreciably increase the permeation of IFN$\alpha$ across mucosal tissue in the *in vitro* model. The combination of IFN$\alpha$, 1% or 2% bile salt and effervescent composition, however, does appears to produce appreciable enhancement of the polypeptide across mucosal tissue. The combination of taurocholate and effervescent formulation together with IFN$\alpha$ appears to produce a permeation result greater than the sum of the individual permeation effects of the bile salt and effervescent composition alone. These results from the ELISA technique generally support and corroborate the results seen from the fluorescent-labeled polypeptide permeation assay described above.

### *In vivo* Testing of Enhanced Buccal Polypeptide Permeation

#### Anaesthetized Dog Model Protocol

Anesthetized dog models were used to evaluate transmucosal permeation of polypeptide compositions prepared in accordance with the invention. Mature mongrel large-breed (15-35 kg) dogs were screened for medical conditions to qualify. For testing, the dogs were anaesthetized, placed on their right or left side, and cannulated via the cephalic vein. A Teflon ring was attached to the buccal mucosa in a horizontal position to form a reservoir. Biologically active polypeptides to be evaluated were deposited, with or without enhancer components, onto the buccal mucosa surrounded by the ring. Depending upon the experiment, active polypeptides were deposited in the form of solution, dry powder or solid dosage form (tablet). Blood samples were taken every 10 minutes for a period of 2 hours, with additional samples taken up to 4 hours depending upon the protocol. The active-containing compositions were removed from the reservoir at 60 minutes and the reservoir rinsed twice.

At 2 hours, the dog is removed from anesthesia and the ring is removed from its buccal mucosa. Following recovery from anesthesia, subsequent blood samples are taken from the awake dogs. Blood samples are allowed to clot, the serum is separated by centrifugation, and the serum is frozen at -20° or -80° C, depending upon the protocol until analyzed for the active polypeptide content.

**Example 14**

A. Desmopressin Oral Transmucosal Permeation

Desmopressin (deamino-Cysl-D-Arg Vasopressin, or D-Arg Vasopresin) was tested for buccal permeation into the blood stream by various administration forms: solution, powder, and compressed tablet. Desmospressin was tested with effervescent composition EF 1 (Example 1A) alone, with bile salt (sodium taurcholate), and with the combination of bile salt and effervescent composition. Desmopressin was additionally tested with the effervescent excipient ingredients (EF 1 and Example 1A), as well as basic effervescent formulation EF 2 (Example 1A).

Preparation of Desmopressin Tablets

Desmopressin-containing tablets for the experiment were prepared by combining 200 mg of effervescent formulation described in Example 1A (EF 1) with 20 mg sodium taurocholate. This mixture was added directly into a vial of lyophilized desmopressin. The vials with the powders were mixed 30 minutes and then compressed into tablets using a Piccola 8 station tablet press fitted with 5/16" round diameter, beveled edge D-size tooling. Powders from individual vials were deposited into each die and compressed by hand at approximately 3 kN compression force. Several test tablets had been prepared containing effervescent composition and bile salt only to ascertain optimal press conditions before preparing the tablets containing the active polypeptide. Finished tablets were stored dessicated at -20° C prior to use in the study.

Tablets prepared accordingly were tested in dogs per protocol described above. When tablets were used in the study, the tablet was placed directly onto the canine buccal mucosa and hydrated over I minute with water. Sequential blood samples were taken and the serum extracted. Desmopressin was measured using LC/MS/MS analysis o the serum samples.

The following table contains the formulation information evaluated and the bioavailability results from the *in vivo* study.

Table 12a Comparative *in vivo* Formulations and Results

| Formulation | DP only (solution) | DP+EF1 (solution) | DP+TC (solution) | DP+EF2 +TC (solution) | DP+EF1+TC (powder) | DP+EF1 +TC (tablet) |
|---|---|---|---|---|---|---|
| Desmopressin | 830 $\mu$g - 415 $\mu$g/ml in 2 ml | 830 $\mu$g - 415 $\mu$g/ml in 2 ml | 830 $\mu$g - 415 $\mu$g/ml in 2 ml | 830 $\mu$g - 415 $\mu$g/ml in 2 ml | 830 $\mu$g | 830 $\mu$g |
| EF 1 | - | 200 mg (powder) | - | - | 200 mg (powder) | 200 mg |
| EF 2 | - | - | - | 93 mg (powder) | - | - |
| TC | - | - | 20 mg (powder) | - | 20 mg (powder) | 20 mg |
| Saline added | 2 ml | 2 ml | 2 ml | 2 ml | 2 ml | 2 ml |
| **Results (Averages)** | | | | | | |
| First detected | None detected | None detected | None detected | 17.5$\pm$5.0 min | 10 min | 10 min |
| Cmax | None detected | None detected | None detected | 9.3$\pm$1.6 ng/ml | 16.0$\pm$1.0 ng/ml | 9.5$\pm$3.1 ng/ml |

(continued)

| Formulation | DP only (solution) | DP+EF1 (solution) | DP+TC (solution) | DP+EF2 +TC (solution) | DP+EF1+TC (powder) | DP+EF1 +TC (tablet) |
|---|---|---|---|---|---|---|
| Tmax | None detected | None detected | None detected | $103\pm15$ min | $75.0\pm7.1$ min | $90.0\pm14.1$ min |
| DP = desmopressin; TC = sodium taurocholate; EF1 = Excipient Formula 1; EF2 = Excipient Formula 2. | | | | | | |

As can be seen from the above data, desmopressin as the biologically active polypeptide in combination with the bile salt at the given concentration, or with the effervescent formulations, does not appear to appreciably increase the permeation of the polypeptide across the mucosal tissue in the *in vivo* model. The combination of bile salt with the effervescent formulation, however, appears to appreciably enhance the transport/permeation of the polypeptide across the mucosal tissue. The combination of taurocholate and effervescent composition with the polypeptide appears to produce a permeation result, whereas none was observed when the individual bile salt or effervescent compositions were used by themselves with desmopressin.

Moreover, although not wishing to be bound by theory, it appears possible that the effervescent formulation is also effective at enhancing desmopressin permeation when used in combination with taurocholic acid and therefore may indicate that that the core effervescent formulation portion (Excipient Formula 2) of the broader effervescent composition (Excipient Formula 1), both described in Example 1A, may be the effective portion for synergy when paired with taurocholate.

B. Salmon Calcitonin Oral Transmucosal Permeation

Salmon calcitonin (s-Ct) was tested for buccal permeation into the blood stream of anesthetized dogs with effervescent compressed tablets containing s-Ct and the bile salt (sodium taurocholate) at 0%, 2.5%, 5.0%, and 10% bile salt. The tablets were prepared using techniques and equipment similar to that described above for desmopressin. The following table contains the formulation information of the tablets evaluated.

Table 12b.

| Formulation | No NaTC | 2.5% NaTC | 5% NaTC | 10% NaTC |
|---|---|---|---|---|
| Salmon calcitonin | 1.00 | 1.00 | 1.00 | 1.00 |
| Sodium taurocholate | 0.00 | 5.00 | 10.00 | 20.00 |
| Sodium chloride | 2.17 | 1.63 | 1.09 | 0.00 |
| Mannitol 60 | 80.83 | 76.37 | 71.91 | 63.00 |
| Sodium bicarbonate | 42.00 | 42.00 | 42.00 | 42.00 |
| Citric acid | 30.00 | 30.00 | 30.00 | 30.00 |
| Sodium carbonate | 20.00 | 20.00 | 20.00 | 20.00 |
| Crospovidone | 20.00 | 20.00 | 20.00 | 20.00 |
| Mg stearate | 4.00 | 4.00 | 4.00 | 4.00 |
| TOTAL | 200.00 | 200.00 | 200.00 | 200.00 |

Tablets were tested in anesthetized dogs using the procedure described for desmopressin in dogs. As the following table demonstrates, there is an improvement in transmucosal permeability of s-Ct in anesthetized dogs when NaTC is added to an effervescent formulation as evidenced by a large increase in AUC (estimated using the trapezoid rule method). Although there are differences in absorption between dogs, the data are consistent for each individual dog. There is a large improvement in permeability by adding 2.5% NaTC or more to the effervescent formulation.

Table 12C. Effect on AUC (pg min/mL) of varying amounts of NaTC on permeability of sCt in effervescent tablets *in vivo* (all tablets include sCt and EF1)

| Dog | No NaTC | 2.5% NaTC | 5% NaTC | 10% NaTC | 10% NaTC repeat |
|-----|---------|-----------|---------|----------|-----------------|
| 90 | 21,000 | | 100,000 | 190,000 | |
| 95 | 11,000 | | 130,000 | 255,000 | |
| 101 | | 80,000 | 200,000 | 300,000 | 320,000 |
| 103 | | 95,000 | 230,000 | 300,000 | 380,000 |

**Example 15**

A. Comparative *in vivo* Insulin (Solution) Permeation Study

Human recombinant insulin was tested for buccal permeation into the blood stream in various compositions in solution applied to canine buccal mucosa. Insulin was tested by itself with effervescent formulation EF 1 (Example 1A), in combination with bile salt and with bile salt and effervescent formulations EF1 and EF2. The procedure used for this study involved a "Glucose Clamp" type protocol, whereby 5% dextrose is infused over time in the amount necessary to maintain blood glucose levels relatively constant. The total amount of dextrose used is recorded as an indirect measure of the amount of functional, biologically active insulin absorbed. Additionally, absorbed insulin was measured directly, with the serum samples tested for insulin levels using ELISA kit (KAQ1251 obtained from Biosource, Camarillo, California). The amount of insulin passing through the buccal tissue was measured over time. Insulin was tested with Excipient Formula 1 (EF 1) as well as the effervescent formulation Excipient Formula 2 (EF 2). The formulations tested and results are summarized in the following table.

Table 13 Comparative *In vivo* Insulin Formulations and Results

| Formulation | In alone (solution) | In+EF1 (solution) | In+EF2 (solution) | In+TC (solution) | In+EF1 +TC (solution) | IN+EF2 +TC (solution) |
|-------------|---------------------|-------------------|-------------------|------------------|-----------------------|-----------------------|
| Insulin | 1 IU/kg/ml | 1 IU/kg/ml | 1 IU/kg/ml | 1 IU/kg/ml | 1 IU/kg/ml | 1 IU/kg/ml |
| EF1 | - | 200 mg (powder) | - | - | 200 mg (powder) | - |
| EF2 | - | - | 93 mg (powder) | - | - | 93 mg (powder) |
| TC | - | - | - | 20 mg (powder) | 20 mg (powder) | 20 mg (powder) |
| Saline | 2 ml | 2 ml | 2 ml | 2 ml | 2 ml | 2 ml |
| **Results (Averages)** | | | | | | |
| In first detected | 60 min. | 20±14 min. | 15±10 min | None detected | 13±5 min. | 10 min. |
| Cmax | 549±776 ng/ml | 524±252 ng/ml | 582±347 ng/ml | None detected | 1814±638 ng/ml | 1754±773 ng/ml |
| Tmax | 80 min | 75±21 min | 70±12 min | None detected | 58±5 min | 60 min |
| Dextrose used | 1164±1435 mg | 235±469 mg | 292±584 mg | 167±18 mg | 7273±4959 mg | 9406±9429 mg |
| In = Insulin; EF 1 = Excipient Formula 1; EF 2 = effervescent composition Excipient Formula 2; TC = sodium taurocholate. | | | | | | |

B. Comparative *in vivo* Insulin (Tablet) Permeation Study

Insulin in powder form was formulated into tablets according to the following formulations:

Table 14 Comparative Insulin Tablet (200 mg) Formulations

| Ingredient: | In only (tablet) | In+EF1 (tablet) | In+TC (tablet) | In+EF1+TC (tablet) |
|---|---|---|---|---|
| Insulin (In) | 2.86 mg | 2.86 mg | 2.86 mg | 2.86 mg |
| Sodium taurocholate (TC) | - | - | 20 mg | 20 mg |
| Mannitol | 173.14 mg | 81.14 mg | 153.14 mg | 61.14 mg |
| Sodium bicarbonate | - | 42 mg | - | 42 mg |
| Citric acid | - | 30 mg | - | 30 mg |
| Sodium carbonate | - | 20 mg | - | 20 mg |
| Crospovidone | 20 mg | 20 mg | 20 mg | 20 mg |
| Magnesium stearate | 4 mg | 4 mg | 4 mg | 4 mg |
| Total: | 200 mg | 200 mg | 200 mg | 200 mg |
| In=Insulin; EF1 = Excipient Formula 1; TC = sodium taurocholate. | | | | |

The tablets were prepared using a techniques and equipment similar to that described above for desmopressin, and the tablets were tested in dogs using the anesthetized dog model protocol described above. For the *in vivo* canine study, the tablets were placed directly onto the buccal mucosa and hydrated over a period of 1 minute with water. Serum samples were collected and analyzed using an ELISA kit, and the data was analyzed and average results obtained. The results are summarized in the following table:

Table 15 *In vivo* Permeability Data for Insulin Tablets (200 mg)

| Formulation | In only (tablet) | In+EF1 (tablet) | In+TC (tablet) | In+EF1+TC (tablet) |
|---|---|---|---|---|
| In first detected | $35\pm21$ min | 10 min | $15\pm7$ min | 10 min |
| Cmax | $428\pm18$ pg/ml | $1260\pm53$ pg/ml | $439\pm204$ pg/ml | $3503\pm1505$ pg/ml |
| Tmax | $90\pm42$ min | $65\pm7$ min | $55\pm7$ min | $67\pm12$ min |
| Dextrose used | 0 | 0 | 0 | $20639\pm7607$ mg |
| In=Insulin; EF1 = Excipient Formula 1; TC = sodium taurocholate. | | | | |

**Example 16** Comparative Bile Salt Data

An experiment was performed in order to determine the most effective bile salts that could work with the invention. The bile salt candidates were initially screened for toxicity levels in cell culture test (*in vitro*). A solution of the bile salts dissolved in Krebs-Ringer buffer solution and successive two-fold dilutions were prepared and tested. The MTS assay for toxicity (readily available to those skilled in the art) was performed on the samples. Using the Tox 50 value obtained, the enhancement capability of bile salts in combination with a defined amount of Excipient Formula 1 powder was determined. Samples were prepared in 2X, 1X, ½ X and ¼ X Tox 50 amounts and evaluated for their permeability enhancement capability alone and in combination with about 5 to 6 μg of the effervescent excipient formulation (Excipient Formula 1) described above. Thus, enhancement capability and synergistic capabilities with the effervescent excipient component compared.

A wide collection of bile salts thus tested were screened for prospective enhancement properties when combined with effervescent excipient formulations within the context of the invention. Accordingly, the following bile salts were selected based on prospective evaluation: sodium taurocholate (TC), sodium glycocholate (GC), sodium glycodeoxycholate (GDC), sodium taurodeoxycholate (TDC), sodium cholate (C), sodium taurochenodeoxycholate (TCDC), and sodium tauroursodeoxycholate (TUDC), and combinations thereof.

Enhancement ratio (ER) is the ratio of the apparent permeability coefficient with the enhancer treatment divided by the apparent permeability coefficient with the active ingredient alone. This technique and calculation was performed for each of the bile salts tested.

The results are shown in Figure 13. As can be seen from the data in the figure, for each of the seven bile salts tested, the combination of the active polypeptide (insulin) in combination with both the bile salt and the effervescent excipient component according to the composition of the invention, appears to produce substantially higher permeability effects as indicated by the ER as compared to either permeation enhancement agent used individually (i.e., bile salt alone or effervescent excipient alone).

As can be seen from the Figure 13, neither formulation using the polypeptide in combination with the bile salt alone, nor the polypeptide with the effervescent excipient component alone, appears to accomplish permeability levels to the extent of the ultimate combination of the polypeptide (insulin) together with both bile salt and effervescent excipient component. This result appeared to be consistently achieved irrespective of which bile salt was used in the formulations.

**Example 17** IGF-1 Analysis by ELISA

*In vitro* permeability was evaluated for Insulin-like Growth Factor-1 (IGF-1). IGF-1 is composed of 70 amino acids with 3 internal disulfide bonds. It has a molecular weight of about 7600 daltons. IGF-1 is a complex growth factor with multiple functions. Using the cell culture model described above, the permeability IGF-1 was tested. IGF-1 was measured by an ELISA kit (DG-100, R&D Systems, Minneapolis MN). Using this technique, IGF-1 is captured by a plastic-bound antibody and developed using an enzyme-linked specific antibody that provides optical density when incubated with the enzyme-specific substrate. Thus, the amount of IGF-1 passing through the cultured buccal cells was measured over time and used to calculate apparent permeability coefficients (Papp).

The results are depicted in Figure 14. As can be seen from the data in the figure, IGF-1 as the active polypeptide alone combined with the bile salt (sodium taurocholate), or the polypeptide combined with the effervescent composition (effervescent Excipient Formula 2 described in Example 1A and referred to as EF 2) alone, does not appear to exhibit an increase in permeation of the polypeptide IGF-1 across mucosal tissue in the *in vitro* model.

The combination of the polypeptide IGF-1, EF2 and 1% bile salt, however, appears to exhibit enhanced permeation/transport of the polypeptide across the mucosal tissue. When used together, the combination of taurocholate and the effervescent excipient formulation with the polypeptide appears to produce a permeation result greater than the sum of the individual permeation effects of the polypeptide with the same bile salt alone and same effervescent formulation alone. These results using an ELISA generally support and corroborate the results obtained using the fluorescent-labeled permeation assay technique described above.

**Example 18** Horseradish Peroxidase Analysis by Enzymatic Activity

Horseradish Peroxidase (HRPO-b) was obtained tagged with biotin (Sigma Chemicals, St. Louis MO). HRPO-b is about 308 amino acids with a molecular weight of about 34,000 daltons. It is a common enzyme used in bioassays such as ELISA. It was purified from other components of the samples using magnetic beads coated with strepavidin. Enzymatic activity of HRPO-b on the washed magnetic beads was measured by analyzing the conversion of peroxidase substrate (R&D Systems, Minneapolis MN) at O.D at 450 nM. Thus, the amount of HRPO-b passing through the cultured buccal cells was measured over time and used to calculate apparent permeability coefficients (Papp).

The results are depicted in Figure 15. As can be seen from the data in the figure, HRPO-b as the active polypeptide by itself, combined with the bile salt (sodium taurocholate) alone, or the polypeptide combined with the effervescent composition (effervescent Excipient Formula 2 described in Example 1A and referred to as EF 2) alone, does not appear to exhibit an increase in permeation of the polypeptide HRPO-b across mucosal tissue in the *in vitro* model.

The combination of the polypeptide HRPO-b, EF2 and 1% bile salt, however, appears to exhibit enhanced permeation/transport of the polypeptide across the mucosal tissue. When used together, the combination of taurocholate and the effervescent excipient formulation with the polypeptide appear to produce a permeation result greater than the sum of the individual permeation effects of the polypeptide with the same bile salt alone and same effervescent formulation alone. These results using intact enzymatic activity generally support and corroborate the results obtained using the fluorescent-labeled permeation assay technique described above.

The experimental data described herein above appears to support a synergistic interaction between the effervescent component ingredients, the bile salt, and biologically active polypeptide ingredients of the compositions of the invention. Although not wishing to be bound by theory, it is believed that the effervescent excipient formulation interacts with the bile salt ingredient in a manner that facilitates the transport of polypeptides across the mucosa as a drug delivery route. Another possible explanation may be that the combination of the individual effects of the effervescent component and the bile salt on mucosal tissue create a greater total net cellular permeability.

**Industrial Applicability**

The invention is useful in delivery of various polypeptides via the oral transmucosal delivery route to recipients. Further, the invention can be used within the medical, pharmaceutical or nutritional fields.

The invention has been described herein above with reference to various and specific embodiments and techniques. It will be understood by one of ordinary skill in the art, however, that reasonable variations and modifications can be made from such embodiments and techniques without significantly departing from the scope of the invention as defined by the following claims.

**Claims**

1.  An oral transmucosal composition comprising:

    a) a therapeutically effective amount of a biologically active polypeptide;
    b) an effervescent excipient component; and
    c) a bile salt.

2.  The composition according to claim 1, wherein the polypeptide has a molecular weight ranging from about 500 Daltons to about 200 kiloDaltons.

3.  The composition according to claim 2, wherein the polypeptide has a molecular weight ranging from about 1000 Daltons to about 20,000 Daltons.

4.  The composition according to claim 1, wherein said effervescent excipient component comprises an acid and a base.

5.  The composition according to claim 4, wherein said effervescent excipient component comprises citric acid and sodium bicarbonate.

6.  The composition according to claim 1, wherein said bile salt is selected from the group consisting of sodium taurocholate, sodium glycocholate, sodium glycodeoxycholate, sodium taurodeoxycholate, sodium cholate, sodium taurochenodeoxycholate, and sodium tauroursodeoxycholate, and combinations thereof.

7.  The composition according to claim 6, wherein said bile salt is sodium taurocholate.

8.  The composition according to claim 1, wherein said effervescent excipient component further comprises a pH adjusting substance.

9.  The composition according to claim 8, wherein the pH adjusting substance is a carbonate.

10. The composition according to claim 9, wherein the pH adjusting substance is sodium carbonate.

11. The transmucosal composition according to claim 1 further comprising a disintegrant.

12. The composition according to claim 11, wherein said disintegrant is a starch glycolate.

13. The composition according to claim 12, wherein said starch glycolate is sodium starch glycolate.

14. The composition according to claim 1 or 11, wherein said biologically active polypeptide is selected from the group consisting of amylin, pink salmon-derived calcitonin (s-CT), glucagon-likepeptide 1 (GLP-1), glucagon, parathyroid hormone (PTH), oxytocin, and desmopressin (D-Arg vasopressin).

15. The composition according to claim 1 or 11, wherein said biologically active polypeptide is selected from the group consisting of insulin, protein YY (PYY), IFN-$\alpha$, IFN-$\beta$, and IFN-$\gamma$.

16. The composition according to any one of claims 1 to 15 for use in a method of enhancing Transmucosal absorption of the biologically active polypeptide.

**17.** A solid oral dosage form comprising an oral transmucosal composition according to claim 1.

**18.** The dosage form according to claim 17, wherein said effervescent excipient component further comprises a pH adjusting substance.

**19.** The dosage form according to claim 17, wherein said polypeptide is insulin.

**20.** An oral transmucosal composition comprising a therapeutically effective amount of insulin, an effervescent excipient component; and a bile salt for use in a method of treating diabetes in a recipient in need thereof.


**Patentansprüche**

**1.** Orale, transmukosale Zusammensetzung, umfassend:

  a) eine therapeutisch wirksame Menge eines biologisch aktiven Polypeptids;
  b) eine Brauseexzipient-Komponente; und
  c) ein Gallensalz.

**2.** Zusammensetzung gemäß Anspruch 1, wobei das Polypeptid ein Molekulargewicht im Bereich von etwa 500 Dalton bis etwa 200 Kilodalton aufweist.

**3.** Zusammensetzung gemäß Anspruch 2, wobei das Polypeptid ein Molekulargewicht im Bereich von etwa 1000 Dalton bis etwa 20.000 Dalton aufweist.

**4.** Zusammensetzung gemäß Anspruch 1, wobei die Brauseexzipient-Komponente eine Säure und eine Base umfasst.

**5.** Zusammensetzung gemäß Anspruch 4, wobei die Brauseexzipient-Komponente Citronensäure und Natriumbicarbonat umfasst.

**6.** Zusammensetzung gemäß Anspruch 1, wobei das Gallensalz ausgewählt ist aus der Gruppe, bestehend aus Natriumtaurocholat, Natriumglycocholat, Natriumglycodesoxycholat, Natriumtaurodesoxycholat, Natriumcholat, Natriumtaurochenodesoxycholat und Natriumtauroursodesoxycholat sowie Kombinationen davon.

**7.** Zusammensetzung gemäß Anspruch 6, wobei das Gallensalz Natriumtaurocholat ist.

**8.** Zusammensetzung gemäß Anspruch 1, wobei die Brauseexzipient-Komponente ferner einen pH-Wertregulierenden Stoff umfasst.

**9.** Zusammensetzung gemäß Anspruch 8, wobei der pH-Wert-regulierende Stoff ein Carbonat ist.

**10.** Zusammensetzung gemäß Anspruch 9, wobei der pH-Wert-regulierende Stoff Natriumcarbonat ist.

**11.** Transmukosale Zusammensetzung gemäß Anspruch 1, ferner umfassend ein Sprengmittel.

**12.** Zusammensetzung gemäß Anspruch 11, wobei das Sprengmittel ein Stärkeglycolat ist.

**13.** Zusammensetzung gemäß Anspruch 12, wobei das Stärkeglycolat Natriumstärkeglycolat ist.

**14.** Zusammensetzung gemäß Anspruch 1 oder 11, wobei das biologisch aktive Polypeptid ausgewählt ist aus der Gruppe, bestehend aus Amylin, Calcitonin aus Buckellachs (s-CT), Glucagon-ähnlichem Peptid-1 (GLP-1), Glucagon, Parathyroidhormon (PTH), Oxytocin und Desmopressin (D-Arg-Vasopressin).

**15.** Zusammensetzung gemäß Anspruch 1 oder 11, wobei das biologisch aktive Polypeptid ausgewählt ist aus der Gruppe, bestehend aus Insulin, Protein YY (PYY), IFN-$\alpha$, IFN-$\beta$ und IFN-$\gamma$.

**16.** Zusammensetzung gemäß einem der Ansprüche 1 bis 15 für die Verwendung bei einem Verfahren zum Verstärken der transmukosalen Absorption des biologisch aktiven Polypeptids.

**17.** Feste orale Dosierungsform, umfassend eine orale, transmukosale Zusammensetzung gemäß Anspruch 1.

**18.** Dosierungsform gemäß Anspruch 17, wobei die Brauseexzipient-Komponente ferner einen pH-Wertregulierenden Stoff umfasst.

**19.** Dosierungsform gemäß Anspruch 17, wobei das Polypeptid Insulin ist.

**20.** Orale, transmukosale Zusammensetzung, umfassend eine therapeutisch wirksame Menge Insulin, eine Brauseexzipient-Komponente und ein Gallensalz, für die Verwendung bei einem Verfahren zum Behandeln von Diabetes bei einem Empfänger mit Bedarf daran.

**Revendications**

**1.** Composition transmuqueuse orale, comprenant :

a) une quantité thérapeutiquement efficace d'un polypeptide biologiquement actif ;
b) un composant excipient effervescent ; et
c) un sel biliaire.

**2.** Composition selon la revendication 1, dans laquelle le polypeptide a une masse moléculaire comprise dans la plage d'environ 500 Daltons à environ 200 kiloDaltons.

**3.** Composition selon la revendication 2, dans laquelle le polypeptide a une masse moléculaire comprise dans la plage d'environ 1000 Daltons à environ 20 000 Daltons.

**4.** Composition selon la revendication 1, dans laquelle ledit composant excipient effervescent comprend un acide et une base.

**5.** Composition selon la revendication 4, dans laquelle ledit composant excipient effervescent comprend de l'acide citrique et du bicarbonate de sodium.

**6.** Composition selon la revendication 1, dans laquelle ledit sel biliaire est choisi dans le groupe consistant en le taurocholate de sodium, le glycocholate de sodium, le glycodésoxycholate de sodium, le taurodésoxycholate de sodium, le cholate de sodium, le taurochénodésoxycholate de sodium et le tauroursodésoxycholate de sodium, et les combinaisons de ceux-ci.

**7.** Composition selon la revendication 6, dans laquelle ledit sel biliaire est le taurocholate de sodium.

**8.** Composition selon la revendication 1, dans laquelle ledit composant excipient effervescent comprend en outre une substance d'ajustement du pH.

**9.** Composition selon la revendication 8, dans laquelle la substance d'ajustement du pH est un carbonate.

**10.** Composition selon la revendication 9, dans laquelle la substance d'ajustement du pH est le carbonate de sodium.

**11.** Composition transmuqueuse selon la revendication 1, comprenant en outre un désintégrant.

**12.** Composition selon la revendication 11, dans laquelle ledit désintégrant est un amidon-glycolate.

**13.** Composition selon la revendication 12, dans laquelle ledit amidon-glycolate est l'amidon-glycolate de sodium.

**14.** Composition selon la revendication 1 ou 11, dans laquelle ledit polypeptide biologiquement actif est choisi dans le groupe consistant en l'amyline, la calcitonine dérivée du saumon rose (s-CT), le glucagon-like peptide 1 (GLP-1), le glucagon, l'hormone parathyroïdienne (PTH), l'oxytocine et la desmopressine (D-Arg vasopressine).

**15.** Composition selon la revendication 1 ou 11, dans laquelle ledit polypeptide biologiquement actif est choisi dans le groupe consistant en l'insuline, la protéine YY (PYY), l'IFN-$\alpha$, l'IFN-$\beta$ et l'IFN-$\gamma$.

**16.** Composition selon l'une quelconque des revendications 1 à 15 pour utilisation dans un procédé pour renforcer l'absorption transmuqueuse du polypeptide biologiquement actif.

**17.** Forme posologique orale solide comprenant une composition transmuqueuse orale selon la revendication 1.

**18.** Forme posologique selon la revendication 17, dans laquelle ledit composant excipient effervescent comprend en outre une substance d'ajustement du pH.

**19.** Forme posologique selon la revendication 17, dans laquelle ledit polypeptide est l'insuline.

**20.** Composition transmuqueuse orale comprenant une quantité thérapeutiquement efficace d'insuline, un composant excipient effervescent ; et un sel biliaire, pour utilisation dans un procédé de traitement du diabète chez un receveur en ayant besoin.

**Average Apparent Permeability Coefficients Based on Calculated Amylin-FAM Donor Concentration**

FIG. 1

Average Apparent Permeability Coefficients Based on Calculated s-Calcitonin-FAM Donor Concentration and Fluorescent Data

FIG. 2

## Average Apparent Permeability Coefficients Based on Calculated GLP-1-FAM Donor Concentration

*FIG. 3*

**Average Apparent Permeability Coefficients Based on Calculated Insulin-FITC Donor Concentration**

FIG. 4

**Average Apparent Permeability Coefficients Based on Calculated Glucagon-FAM Donor Concentration**

*FIG. 5*

FIG. 6

**Average Apparent Permeability Coefficients Based on Calculated Oxytocin-FAM Donor Concentration**

FIG.7

EP 2 155 229 B1

# Average Apparent Permeability Coefficients Based on Calculated AVP-FAM Donor Concentration

*FIG. 8*

**Average Apparent Permeability Coefficients Based on Calculated Peptide YY-FAM Donor Concentration**

FIG.9

**Average Apparent Permeability Coefficients Based on Calculated s-Calcitonin Donor Concentration**

FIG. 10

FIG.11

Average Apparent Permeability Coefficients Based on Calculated Hu-IFN-α2b Donor Concentration

FIG.12

*FIG. 13*

**Average Apparent Permeability Coefficients Based on Calculated IGF-1 Donor Concentration - (SEM Bars) -**

**FIG. 14**

EP 2 155 229 B1

**Average Apparent Permeability Coefficients Based on Calculated HRPO-b Donor Concentration**

FIG. 15

EP 2 155 229 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5725852 A, Igari
- US 5482706 A
- US 6210699 B, Acharya
- US 5238917 A, Fujii
- US 5863555 A, Heiber
- US 5766620 A
- US 6200604 B
- US 6974590 B
- US 20050169989 A
- US 1026132 A

- US 20050142197 A
- US 1026327 A
- US 20050142198 A
- US 1027353 A
- US 20050163838 A
- US 11026759 B
- US 5178878 A
- US 5503846 A
- US 6155423 A
- US 6024981 A